(19) Europäisches Patentamt
European Patent Office
Office européen des brevets

(11) Publication number: **0 272 580 B1**

# (12) EUROPEAN PATENT SPECIFICATION

(45) Date of publication of patent specification: **17.08.94**

(51) Int. Cl.$^5$: **C09K 19/30**, C07C 69/74, C07C 205/06, C07C 49/813, C07C 25/18, C07C 211/00, C07C 331/28

(21) Application number: **87118518.7**

(22) Date of filing: **14.12.87**

## (54) Liquidcrystalline cyclohexylbenzene derivatives their manufacturing and novel nematic admixtures containing same.

(30) Priority: **24.12.86 PL 263243**

(43) Date of publication of application:
**29.06.88 Bulletin 88/26**

(45) Publication of the grant of the patent:
**17.08.94 Bulletin 94/33**

(84) Designated Contracting States:
**CH DE GB LI**

(56) References cited:
**EP-A- 0 102 047**
**EP-A- 0 129 177**
**EP-A- 0 144 648**
**EP-A- 0 227 004**

**CHEMICAL ABSTRACT, vol. 103, no. 17, 28 October 1985, Columbus, Ohio, US, p. 691, no. 141633r; & JP-A-60 41 638**

**CHEMICAL ABSTRACT, vol. 101, no. 9, 27 August 1984, Columbus, Ohio, US, p. 617, no. 72447c; & JP-A-59 29 640**

(73) Proprietor: **Wojskowa Akademia Techniczna im. Jaroslawa Dabrowskiego**

**PL-01-489 Warszawa-49 (PL)**

(72) Inventor: **Dabrowski, Roman**
**ul. Buska 40**
**02-924 Warszawa (PL)**
Inventor: **Szczucinski, Tomasz**
**ul. Rozlogi 9 m. 4**
**01-310 Warszawa (PL)**
Inventor: **Dziaduszek, Jerzy**
**ul. Mlynarska 30a m. 26**
**01-171 Warszawa (PL)**

(74) Representative: **von Füner, Alexander, Dr. et al**
**Patentanwälte v. Füner, Ebbinghaus, Finck,**
**Postfach 95 01 60**
**D-81517 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Field of the invention

The present invention concerns naval cyclohexylbenzene derivatives and liquid crystals admixtures containing same applicable in electro-optical devices as dielectrics. The invention also concerns methods of manufacturing these liquid crystal coumpounds

Description of the prior art

Liquid crystals have gained in the recent years great importance as dielectrics in electro-optical displays thanks to that their optical properties may be easily influenced by an electric field. Many types of such devices are widely known and they can be based on various electro-optical effects such as for instance,dynamic scattering, twisted nematic effect, cholesteric-nematic phase transition. Liquid crystal displays are ever more widely used not only at practically constant indoor temperatures, but also at temperatures varying in a wide range. The latter application concern main outdoor use in such devices as parkometers, gasoline pump stations, car dashboards, portable measuring instruments etc. The quoted devices require liquid crystal admixtures of parameters higher as compared with these operating indoors at practically constant temperatures. These mixtures should reveal the nematic phase in the temperature range of -20 to +80°C or even wider, low viscosity also in the lower temperature region (since viscosity limit the switching on and off times). Under outdoor conditions the intensity of UV radiation and humidity also vary in a wide range, so the material used should be resistant to light and water.

No such single liquid crystal compound exists that would. fulfil all the requirements laid before the liquid crystalline material, especially when the later is used outdoors. Attempts are therefore made to optimize the properties of such materials for given application by mixing many components. According to the present state of art a material with the required properties can be obtained by blending several or even a dozen or so different compounds. To the liquid crystal mixtures whose components reveal nematic or sometimes smectic properties also non-liquidcrystalline and or optically active compunds and dichroic dyes may be added. It is important, however, that these components enter no chemical reactions with each other and that they mutually readily dissolve. The mixture should also reveal a low solid-to-nematic transition point ($T_{Cr \rightarrow N}$), high nematic-to-isotropic liquid transition point ($T_{N \rightarrow I}$), and low viscosity.

An admixture of suitably selected compounds meets better the requirements to be fulfilled by the liquid crystal material for a given application. To obtain liquid crystal admixtures with a wide range of the nematic phase, the components are chosen so that a base admixture of mesogenes with possibly low melting points and low melting enthalpies is prepared.To such admixtures 5 to 40% of tri-or tetranuclear compounds with high clearing points are added. For obtaining base mixtures with low viscosity most suitable are binuclear compounds - derivatives of cyclohexylbenzene - such as described in U.S. Patent 4.528.116 4-(trans-4-alkylcyclohexyl)-1-isothiocyanatobenzenes or in G.F.R. Patent 2.638.864 4-(trans-4-n-alkylcyclohexyl)-1-cyanobenzenes.

A liquid crystal admixture composed of 4-(trans-4-n-alkyl cyclohexyl)-1-isothiocyanatobenzenes only has a viscosity of about 10 mPa s and clearing point below 45°C, besides, it reveals a low value of the elastic constant ratio $k_{33}/k_{11}$ close to unity.A liquid crystal mixture composed of 4-(trans-4-n-alkylcyclohexyl)-1-cyanobenzenes reveals viscosity of about 20 mPa s, clearing point of about 55°C, and high elastic constant ratio $k_{33}/k_{11}$ of about 2. The mixtures obtained of 4-(trans-4-n-alkylcyclohexyl)-1-cyanobenzenes or 4-(trans-4-n-alkylcyclohexyl)-1-isothiocyanatobenzenes only still have clearing points too low for their practical application. Cyano derivatives of terphenyl, cyclohexylbiphenyl or diphenylpyrimidine, or else esters of cyclohexylbenzoic or biphenyl acids are so far the most frequently used compounds for raising the clearing points. The above mentioned trinuclear compounds raise the clearing points of mixtures but at the same time also raise significantly their viscosities. For instance the mixture described in the USSR Patent 738.516 of the composition in wt %.

| | |
|---|---|
| 4-(trans-4-n-propylcyclohexyl)-1-cyanobenzene | 36.1 |
| 4-(trans-4-n-pentylcyclohexyl)-1-cyanobenzene | 30.8 |
| 4-(trans-4-n-heptylcyclohexyl)-1-cyanobenzene | 21.1 |
| 2-(4-cyanophenyl)-5-(4-pentylphenyl)-pyrimidine | 12.0 |

reveals a clearing point of 81 °C, and viscosity of 35 mPa s. Besides, the trinuclear compounds often show low solubility in mixtures of binuclear liquid crystal compounds mostly due to the fairly high melting enthalpies and fairly high melting points. For this reason it happens quite often that these compounds precipitate as solid from liquid crystal admixtures after a certain time of storage of the latter at low temperatures. This is particularly often observed in case of admixtures of low viscosity containing trinuclear compounds in quantities exceeding the eutectic composition.

Summary of the invention

The present invention relates to the use of a new class of multi ring compounds of high chemical stability and excellent solubility which have the ability to extend the nematic range of the base mixture composed of binuclear compounds without significant increase of viscosity. The liquid crystal admixtures containing these new compounds reveal a small temperature dependence of viscosity and threshold voltage. Thanks to this, the performance of the liquid crystal display is improved and its control is simplified.

In "Chemical Abstracts, Vol. 103, No. 17, 28th October 1985, page 691, abstract No. 141633r", trans-4'-(4'-substituted phenyl)cyclohexyl 4-substituted benzoates of the formula

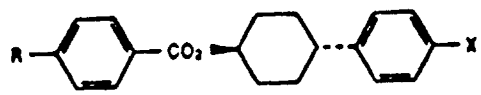

I

are described, wherein R, X = n-$C_5H_{11}$, Cl; Me, Cl; n-$C_8H_{17}$, Cl; MeO, Cl; n-$C_6H_{13}O$, Cl; cyano, Cl; n-$C_5H_{11}$, F, which were prepared and used as liquid crystals having a clearing point from high nematic phase to isotropic liquid phase. Thus, reaction of PhCl, $AlCl_3$, MeCOCl, and cyclohexene 3 h at max. -20° gave 12% trans-4-(p-chlorophenyl)-1-acetylcyclohexane (II). Oxidation of II with 4-$ClC_6H_4C(O)OOH$ in $CHCl_3$ overnight gave 68% trans-4-(p-chlorophenyl)-1-acetyloxycyclohexane (III). Reduction of III with $LiAlH_4$ in $Et_2O$ 2 h at 0° to room temperature gave 77% trans-4-(p-chlorophenyl)cyclohexanol (IV). Esterification of 4-n-$C_5H_{11}C_6H_4COCl$ with IV in $Et_2O$ containing pyridine 1 h at room temperature gave 69% I (R = n-$C_5H_{11}$, X = Cl).

In "Chemical Abstracts, Vol. 101, No. 9, 27th August 1984, page 617, abstract No. 72557c" 4-substituted phenyl trans-4-(4-halophenyl)cyclohexanecarboxylates of the formula

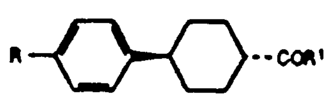

I

are described, wherein R = halo; $R^1$ = p-$R^2C_6H_4O$ where $R^2$ Cl, alkyl, alkoxy, cyano, useful as liquid crystals, which were prepared by esterification of acid chlorides I ($R^1$ = Cl) with p-$R^2C_6H^4OH$. Thus addition of 473 g AcCl and 555 g cyclohexene to a mixture of 1600 g PhCl and 1080 g $AcCl_3$ at -20° over 3 h followed by warming the resulting mixture to room temperature gave 12% I (R = Cl, $R^1$ = Me), treatment of which (165 g) in dioxane with aq. NaOBr (prepared from 385 g Br, 264 g NaOH, and $H_2O$) at room temperature for 2 h gave 160 g I (R = Cl, $R^1$ = OH), the acid chloride (prepared from 160 g of the acid and $SO_2Cl$) in $Et_2O$ containing pyridine was tretaed with 1.64 g p-$Me(CH_2)_4C_6H_4OH$ at room temperature for 1 h to give 77% I, wherein R = Cl, $R^1$ = $Me(CH_2)_4$.

EP 0 144 648 refers to compunds of the formula

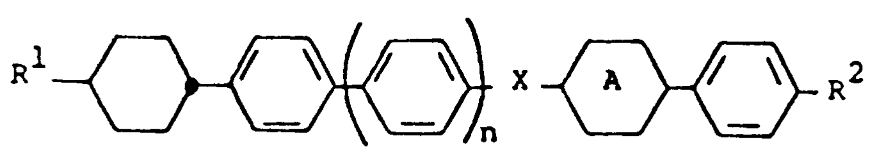

I

wherein n stands for 1 and X for the group -COO-, -OOC- or -$CH_2CH_2$-, or n stands for 0 and X for the group -$CH_2CH_2$-; ring A denotes p-phenylene or trans-1,4-cyclohexylene; $R^2$ represents cyano, p-

cyanophenyl or, when X stands for -COO- or -OOC-, also p-$R^3$-phenyl or trans-4-$R^3$-cyclohexyl; and $R^1$ and $R^3$ are straight-chain $C_1$-$C_{10}$ alkyl, their preparation and use for electrooptical purposes and in gaschromatography.

Detailed description of the invention

The present invention concerns liquid crystalline compounds of the general formula I:

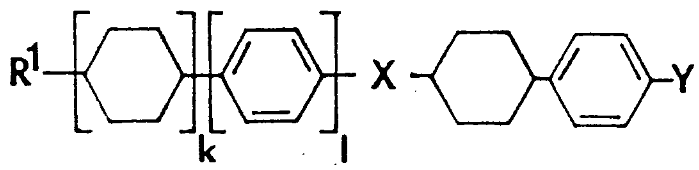

I.

and mixtures containing them. In formula I wherein $R^1$ is a normal or branched alkyl group $H_{2n+1}C_n$- or a normal or branched alkoxy group $H_{2n+1}C_nO$-, n being an integer number from 1 to 12; X is a bridging group from the set: -COO-, -OCO-, $-CHOHCH_2-$, $-CH_2CH_2-$, $-CHOCH_3CH_2-$; Y is a terminal group from the set: $-NO_2$, $-NH_2$, -NCS, the cyclohexane rings are trans-1,4-disubstituted ones and the benzene rings are 1,4-disubstituted ones, and besides one of them may contain sometimes additionally a fluorine atom in position 2 or 3; k and l are integer numbers from the set: 0, 1, 2 fulfilling the condition that k + l = 2 or 3; besides, if X denotes the -COO- or group -OCO- then k + l also may assume the value of 1 ( k = 1 and l = 0 or k = 0 l = 1 ).

By normal or branched alkyl groups $C_nH_{2n+1}$- we understand alkyl chains including 1 to 12 carbon atoms such as methyl, ethyl, propyl, isopropyl, butyl, 2-methylbutyl, pentyl, 3-methylpentyl, 4-methylhexyl, hexyl, heptyl, octyl, nonyl, decyl, undecyl and dodecyl ones. By unbranched or branched alkoxy group $C_nH_{2n+1}O$- we understand an alkyloxy chain in which alkyl portion has the above given meaning.

For comparison the compounds of formula 1, wherein Y is a fluorine, chlorine or bromine atoms are described.

To allow a better description of the compounds that can be derived from the general formula I, or are their intermediates and especially to make possible a short notation of the particularly preferred structures of compound I in a simplified form Arabic numbers and lower case as well as capital letters in addition to Roman numbers have been used additionally. Lower case letters are used to denote the meaning k and m in the formule of compounds with:

| | |
|---|---|
| a k = 1 and l = 0<br>b k = o and l = 1<br>c k = 1 and l = 1<br>d k = 0 and l = 2 | e k = 2 and l = 0 ,<br>f k = 1 and l = 2,<br>g k = 2 and l = 1, |

Arabic numbers are assigned to the central groups as follows:

| | |
|---|---|
| 1 $-COCH_2$<br>2 $-CHOHCH_2$<br>3 -CH = CH-<br>4 $-CH_2CH_2-$,<br>5 $-CHClCH_2-$<br>6 $-CHCN-CH_2-$,<br>7 $-CHBr-CH_2-$, | 8 $-CHF-CH_2-$<br>9 $-CHOCH_3CH_2$<br>10 $-CH_2O-$<br>11 $-OCH_2-$<br>12 -COO-<br>13 -OCO- |

Capital letters are assigned to the terminal groups as follows:

| | |
|---|---|
| A $-NO_2$<br>B $-NH_2$<br>C -NCS | D -F<br>E -Cl<br>F -Br |

According to the adopted notation the compound denoted as I.c4C has the following structural formula:

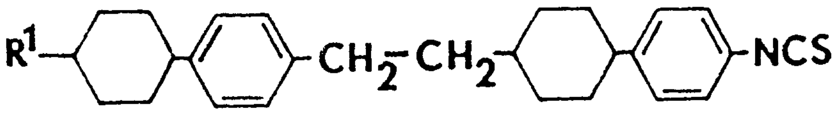

I.c4C

If in the abbreviated notation any of the above given additional symbols is missing, this means that the relevant elements has the same meaning as in the general formula I. According to the abbreviated notation the length of an alkyl chain is defined by adding $C_nH_{2n+1}$ as an prefix. The abbreviated notation will be then in the form $C_nH_{2n+1}$-I or $C_nH_{2n+1}$O-I e.g. $C_3H_7$-I.

From the general formula I we can show groups of compounds with the preferred properties for the following values of the indicators k and l.

For k = 1 and l = 0 the compunds are of the general formula Ia:

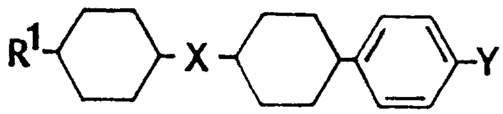

I.a

wherein $R^1$ is a normal or branched alkyl chain with n varying from 1 to 12, preferably from 4 to 10, X is the bridging group -COO- or -OCO-; Y the terminal group -NCS or $-NO_2$

For k = 0 and l = 1 the compounds are of the general formula Ib:

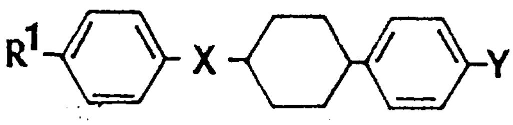

I.b

wherein $R^1$ is a normal or branched alkyl chain or a normal or branched alkoxy group $C_nH_{2n+1}O$- with n varying from 1 to 12, preferably from 4 to 10, X is the bridging group -COO- or -OCO-; Y is the terminal group $-NO_2$ or -NCS

For k = 1 and l = 1 the compounds are of the general formula Ic:

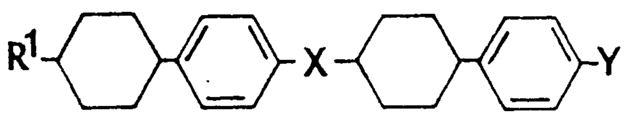

I.c

wherein $R^1$ is a normal or branched alkyl chain with n varying from 1 to 12, preferably from 4 to 10; X is the bridging group from the set: $-CHOHCH_2$-,$-CH_2CH_2$-, $-CHOCH_3CH_2$-; Y is the terminal group from the set: $-NO_2$, $-NH_2$, -NCS, preferably -NCS

For k = 0 and l = 2 the compounds are of the general formula Id:

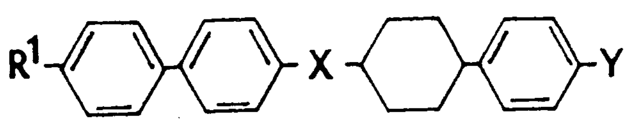

I.d

wherein $R^1$ is a normal or branched alkyl chain with n varying from 1 to 12, preferably from 4 to 8; X is the bridging group from the set: $-CHOHCH_2$- , $-CH_2CH_2$-, $-CHOCH_3CH_2$; Y is the terminal group from the set: $NO_2$, -NCS,

For k = 2 and l = 0 the compounds are of the general formula Ie:

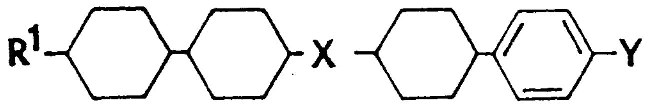

I.e

wherein $R^1$ is a normal alkyl chain with n varying from 1 to 12, preferably from 4 to 10, X is the bridging group from the set: $-CHOHCH_2-$, $-CH_2CH_2-$, $-CHOCH_3CH_2-$, preferably $-CH_2CH_2-$ or, $-CHOCH_3CH_2-$; Y is a terminal group from the set: $-NO_2$, -NCS, $-NH_2$, preferably -NCS.

For k = 1 and l = 2 the compounds are of the general formula If:

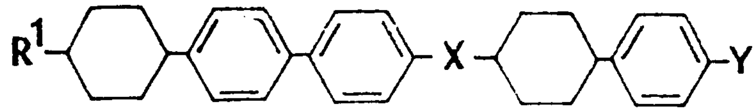

I.f

wherein $R^1$ is a normal alkyl chain with n varying from 1 to 12, preferably from 6 to 10; X is a bridging group from the set: $-CH_2CH_2-$, Y is the terminal group -NCS.

For k = 2 and l = 1 the compounds are of the general formula Ig:

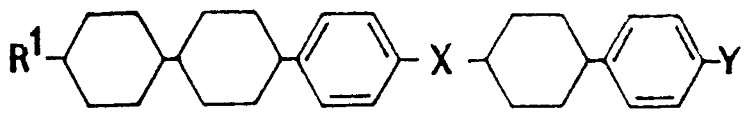

I.g

wherein $R^1$ is a normal or branched alkyl chain with n varying from 1 to 12; X is a bridging group from the set: $-CH_2CH_2-$, $-CHOCH_3CH_2-$, preferably $-CH_2CH_2-$; Y is the terminal group -NCS

Manufacturing of compound I needs substrates which are not commercial attainabled and they should be previously prepared. The compounds of the general formula IIa are convinient substrates for synthesis of the compounds of our invention and also for synthesis of other compounds of a similar structure which are used for comparison.

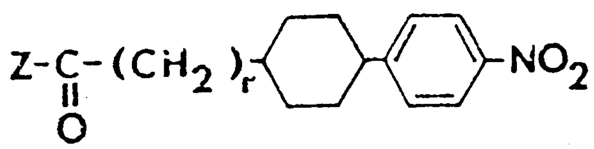

II.A

In IIa Z is a hydroxy group or halogen, preferably chlorine or bromine, r is zero or unity, and wherein the cyclohexane ring is a trans-1,4-disubstituted one, and the benzene ring is 1,4-disubstituted having sometimes additionally a fluorine atom in position 2 or 3.

In schemes 1 and 2 some preferred methods of production of compounds II.A and use of them for obtaining other intermediates for preparation of liquid crystals are given.

The crucial problem of the synthesis of compounds II.A shown in scheme 1 consists in the separation of the orto- and para-nitroesters of compound VII (in which $R^2$ is a fragment of the alcohol used for the esterification of acid V). They can he separated by crystallization. The initial trans-4-phenylcyclohexanecarboxylic (formula V, r = 0) and trans-4-phenylcyclohexylacetic (formula V, r = 1) acids are easily obtained from trans-4-acetylcyclohexylbenzene in the haloform and Willgerodt reaction, respectively.

Scheme 1

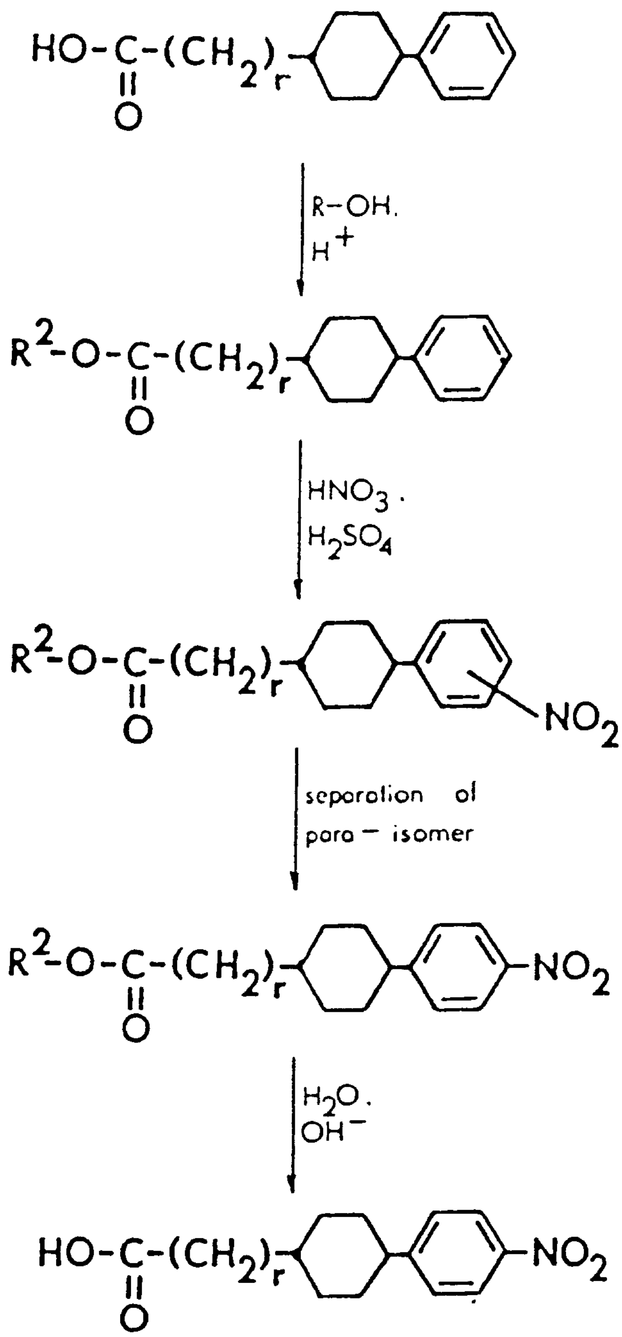


V.

VI.

VII.

VIII.

11.A

Scheme 2

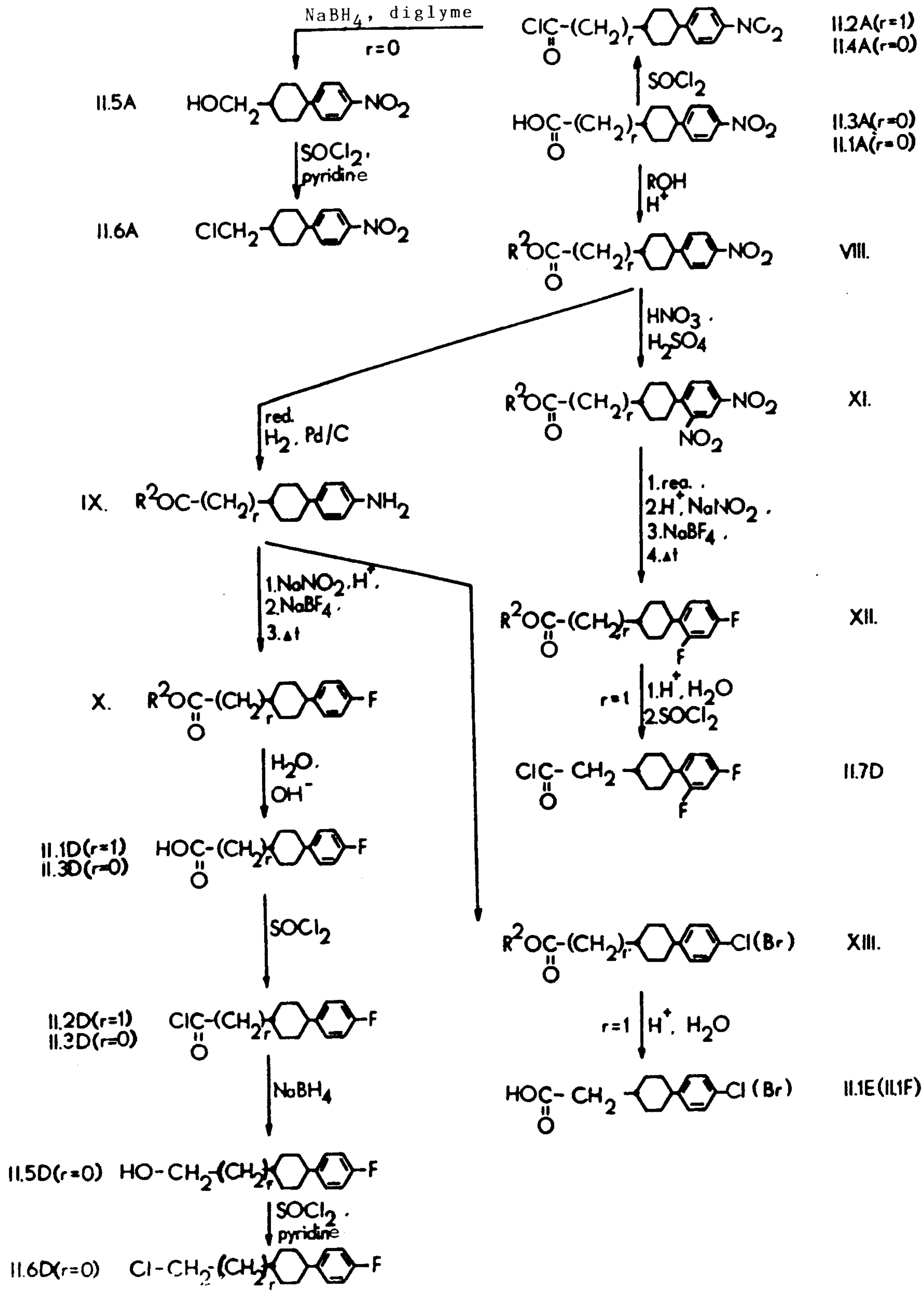


II.2A(r=1) II.4A(r=0)

II.5A

II.3A(r=0) II.1A(r=0)

II.6A

VIII.

XI.

IX.

XII.

X.

II.7D

II.1D(r=1) II.3D(r=0)

XIII.

II.2D(r=1) II.3D(r=0)

II.1E(II.1F)

II.5D(r=0)

II.6D(r=0)

trans-4-Acetylcyclohexylbenzene is produced as described in Polish Patent 128609.

The preferred method of producing new compounds of the general formula I is shown in scheme 3. The hydrocarbon of general formula III, wherein $R^1$, k and l have the same meaning as in formula I, is acylated with the acid halide of the formula II.A where Z is a bromine atom or preferably a chlorine one, and the compound I.1A is obtained.

The reaction of the hydrocarbon of formula III, which for example is trans-4-alkylcyclohexylbenzene or trans-4-n-alkylcyclohexylbiphenyl or trans-4-n-alkylbicyclohexlbenzene or 4-n-alkylbiphenyl or 4-n-alkoxybiphenyl or 4-(2-methylbutyl)biphenyl, and the acid halide of the formula II.A, which is 4-trans-(4-nitrophenyl)cyclohexylacetic chloride ( if 4-trans-(4-chlorophenyl)cyclohexylacetic chloride or 4-trans-(4-fluorophenyl)cyclohexylacetic chloride or 4-trans-(4-bromophenyl-)cyclohexylacetic chloride is used, then compounds analogous to compound I.1A are obtained) is conducted in an organic solvent, chosen from alkanes, chloroalkanes, nitroalkanes, in the presence of aluminum chloride,

Scheme 3

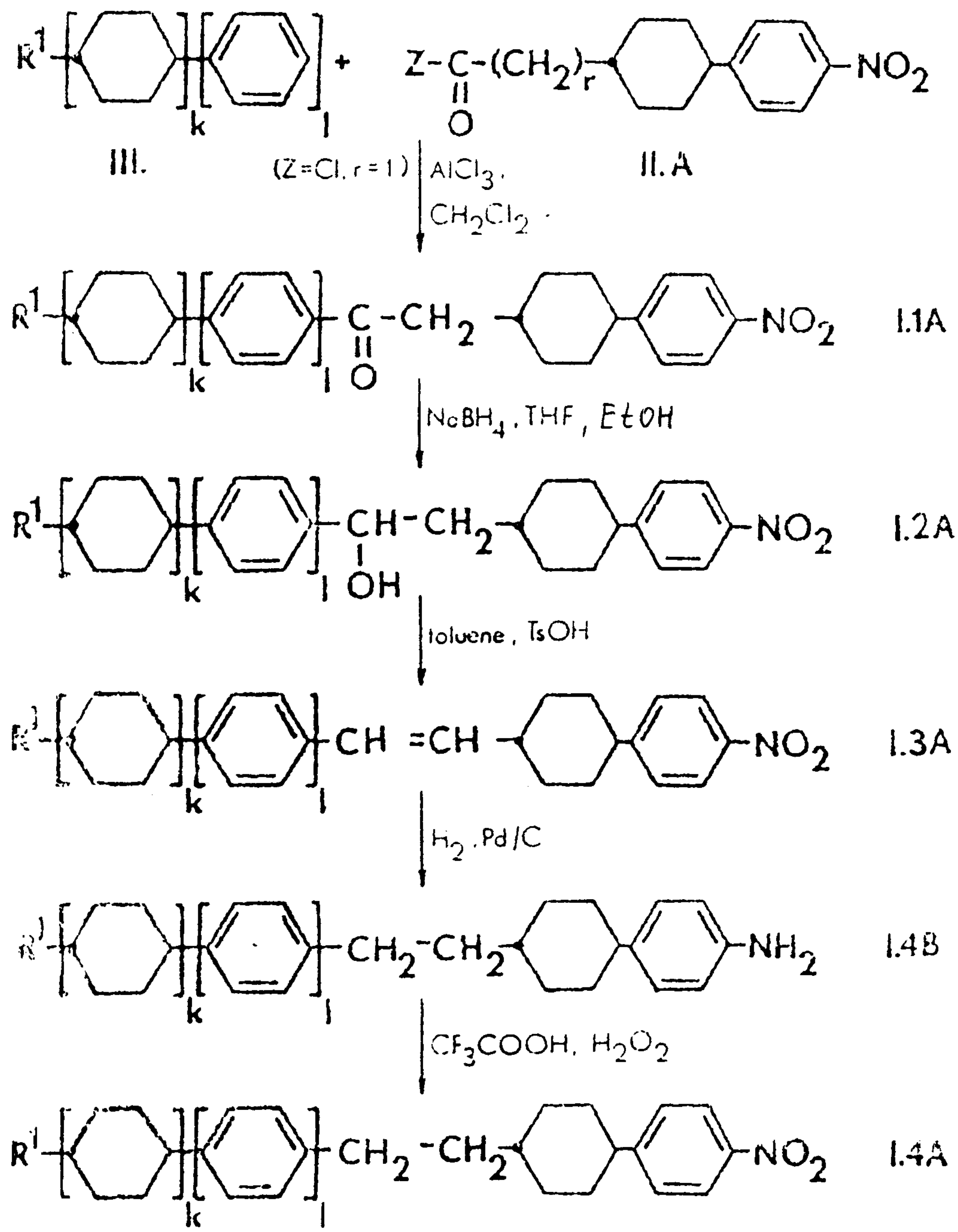

the temperature being kept in the range -20° to +10°C, preferably in the range -5° to +5°C. Among the enumerated solvents methylene chloride, ethylene chloride,chloroform or carbon tetrachloride are recommended in view of the convenience in carrying out further operations connected with the separation and purification of the product.

The recommended procedure is as follows. Solid $AlCl_3$ is added to, e.g., methylene chloride and cooled to below 5°C upon which the solution of cycloalkaneacetic acid chloride of formula II.A in methylene chloride is added dropwise and next the compound of Formula III is added, also dropwise. The contents are stirred for several hours and poured onto a mixture of ice and hydrochloric acid in order to decompose the aluminum chloride complexes. Next generally known methods are applied to separate and purify the reaction product which is the keto compound of the general formula I.1A where the notation is the same as in formula I.

The keto compound of formula I.1A is the basic substrate from which the remaining compounds of the general formula I differing as regards the bridging group X are obtained. Thus the keto compound of the formula I.1A is converted by reduction with sodium borohydride into the compound of the general Formula 1.2A, where the notation is the same as in the general Formula I.

The use of sodium borohydride ($NaBH_4$) as reducing agent is justified by that it permits the reduction of the carbonyl group without affecting other substituents in the terminal position of the molecule liable to reduction; this relates especially to the nitro group. The compound of the general formula I.2A is after purification the final product or substrate for further reactions.

If the compound of the general formula I.2A or I.2 is heated in the presence of an organic solvant preferably toluene, and an acid, preferably p-toluenesulfonic acid, than its dehydration takes place and it converts to the ethylene derivative of the general formula I.3A or I.3 (where the notation is the same as in the General formula I) which after purification is the final product or which is further converted by reduction with hydrogen in the presence of a palladium catalyst to the compound with the bridging group $-CH_2CH_2-$ of formula I.4B.

The terminal group $-NO_2$, reduces together with the double bond in the bridging group to yield compound I.4B, so in order to reproduce it, compound I.4B is oxidized with hydrogen peroxide in trifluoroacetic acid or is converted to the diazonium salt by the known method and next the diazonium group is substituted by the $-NO_2$ group in reaction with sodium nitrite in the presence of cuprous oxide and cuprous sulfate.

It is advantageous to produce the compounds including ester group -COO- or -OCO- as the bridging groups X shown in schemes 4 and 5. The procedure consists in that the compound with alcohol or phenol properties of the general formula IV, where p is equal 1 or zero, and the remaining notation is the same as in the general formula I, is esterified with a compound being an acid or, acid chloride of the general formula II.3 or II.4, or else compound XV is esterified with an easily available acid or its chloride of the formula XIV; in the latter case it is advantageous to use pyridine as catalyst. The product thus obtained is next separated and purified according to the known methods, preferably by crystallization.

Scheme 4

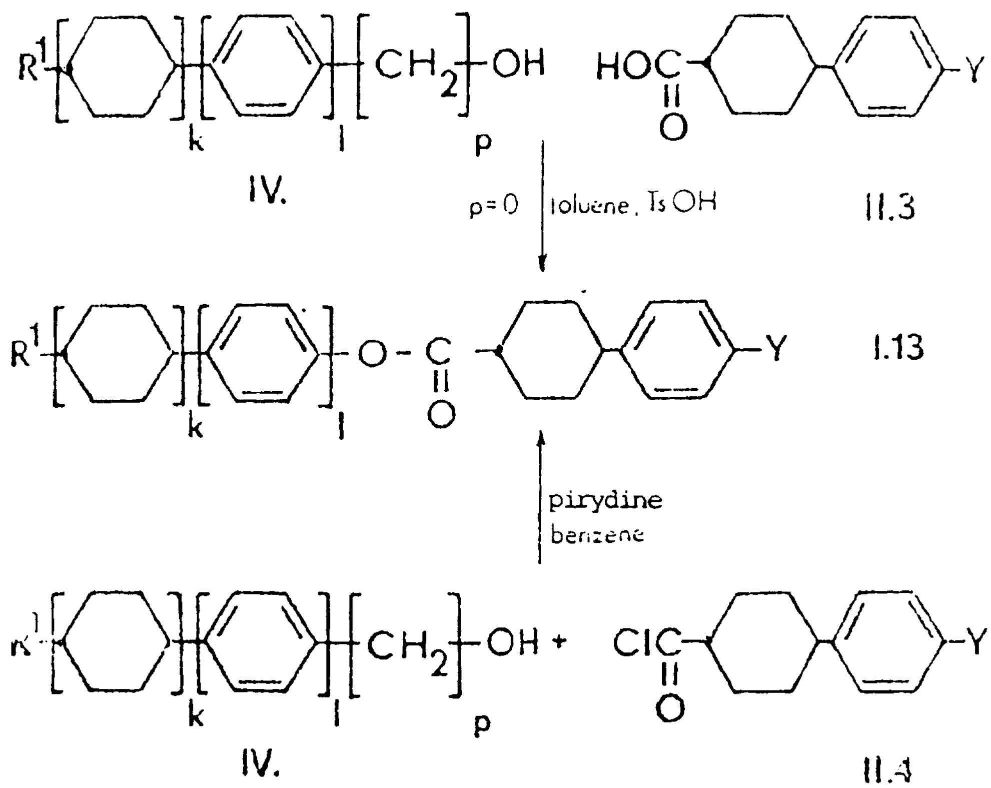


IV. II.3

I.13

IV. II.4

Scheme 5

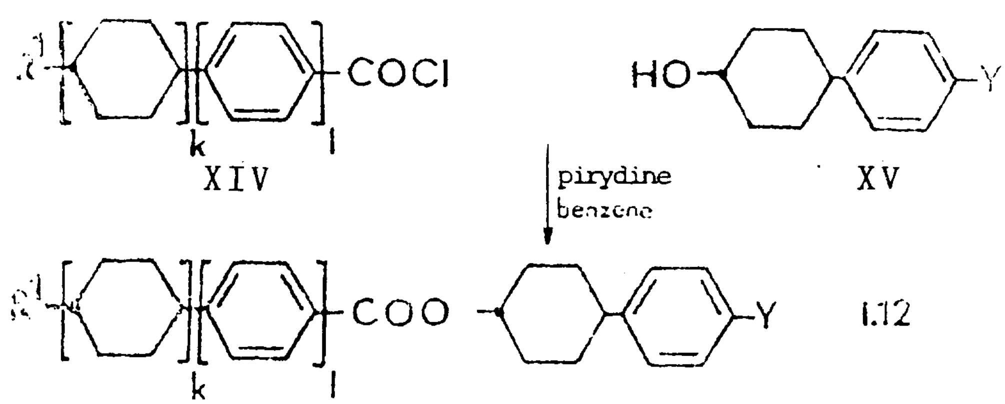


XIV XV

I.12

The compound of general formula I with the terminal group $-NO_2$ and different central groups produced according to Scheme 3 can be easily converted to compounds with other kinds of terminal groups reserved for compound I. This is illustrated in Scheme 6.

Scheme 6.

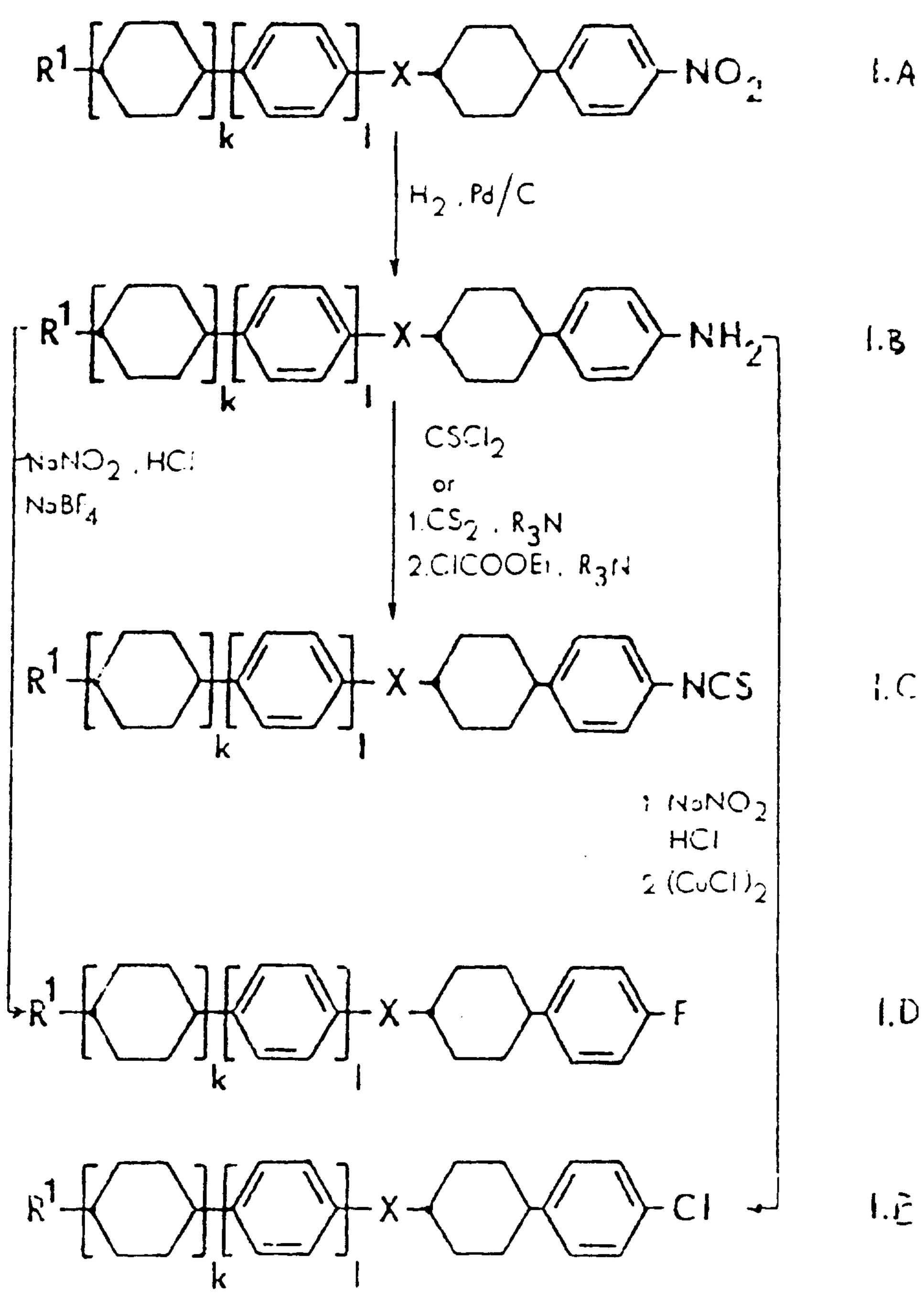


Such a way of proceeding is particularly recommended if we want to obtain compounds I.B or I.C, i.e. compounds with a $-NH_2$ or -NCS terminal groups. Via the compound with the nitro group one can easily obtain compounds with a $-NH_2$ or -NCS terminal group for any central group X by suitably selecting conditions of reduction of the nitro group so that the central group X remains unaffected.

If the bridging group X is one from the set: -COO-, compound I is reduced with hydrogen over palladium; if X is $-COCH_2-$ or $-CHOHCH_2-$ compound I is reduced with iron in the presence of hydrochloric

acid. If X is -CH=CH- the compound is reduced with iron in the presence of hydrochloric acid in order to preserve the bridging group -CH=CH- or, it is reduced with hydrogen over palladium to reduce the bridging group -CH=CH- to $-CH_2CH_2-$.

The amine of the general Formula I.B obtained as a result of reduction is separated and purified by known methods upon which

(a) it is dissolved in an organic solvent, preferably chloroform, and the obtained solution is added dropwise to the mixture containing tiophosgene, water, calcium carbonate and the organic solvent, or

(b) it is dissolved in a hydrocarbon type solvent and triethylamine and carbon disulphide is added to the solution. The crystallizing dithiocarbamic acid salt is separated and subsequently dissolved in an chlorinated hydrocarbon solvent. Triethylamine and alkyl chloroformate are next added to the obtained solution.

After the reaction is completed, the contents are washed with acid and water, dried and isothiocyanate of the general formula I.C is separated.

The following procedure may also be applied. The amine of the general formula I.B is dissolved in a solvent of ether type, preferably in diethyl ether or tetrahydrofurane, upon which dicyclohexylcarbodiimide and carbon disulphide are added to the solution. The obtained isothiocyanate of the formula I.C is separated from dicyclohexylthiourea and purified by known methods.

The compounds of the general formula I which are the object of the invention reveal unique liquid crystal properties.Particular attention should be drawn to their unusually strong nematogenic properties for different kinds of central groups. Nematic properties are revealed not only by the compounds with the -NCS or -F terminal group but also by those with the $-NO_2$ or $-NH_2$ group what is very seldom observed or with hydroxy group in the bridging group such as $-CHOHCH_2$. The nematic phases are observed for long alkyl chains. The above is illustrated in Table 1.

In the table I the following notation is used: Cr denotes the crystalline phase, S-the smectic one, S-the smectic B one, N-the nematic one, I-the isotropic one; the temperature is given in centigrades.

From the above mentioned compounds those are preferred which have general formula I in which k=1 and l=1, and Y=-NCS, and $X=-CH_2CH_2-$; they have low melting points, low viscosities, and low melting enthalpies what ensures their high solublity in other liquid crystal compounds; particulary advantageous

Table 1

| Lp | $R^1$ | k | l | X | Y | phase trans. temperatures | abbreviated notation |
|---|---|---|---|---|---|---|---|
| 1 | $C_5H_{11}$ | 1 | 0 | -OCO- | -F | Cr43N111I | $C_5H_{11}$-I.a13D |
| 2 | $C_5H_{11}$ | 1 | 0 | -OCO- | $-NO_2$ | Cr51N150I | $C_5H_{11}$-I.a13A |
| 3 | $C_5H_{11}$ | 1 | 0 | -OCO- | -NCS | Cr78N180I | $C_5H_{11}$-I.a13C |
| 4 | $C_4H_9$ | 0 | 1 | -OCO- | $-NO_2$ | Cr114N150I | $C_4H_9$-I.b13A |
| 5 | $C_4H_9$ | 0 | 1 | -OCO- | $-NH_2$ | Cr107N124I | $C_4H_9$-I.b13B |
| 6 | $C_4H_9$ | 0 | 1 | -OCO- | -NCS | Cr107N189I | $C_4H_9$-I.b13C |
| 7 | $C_4H_9$ | 0 | 1 | -OCO- | -F | Cr94N116I | $C_4H_9$-I.b13D |
| 8 | $C_4H_9$ | 0 | 2 | $-COCH_2-$ | $-NO_2$ | Cr152N219I | $C_9H_9$-I.d1A |
| 9 | $C_4H_9$ | 0 | 2 | $-CHOHCH_2-$ | $-NO_2$ | Cr135N226I | $C_4H_9$-I.d2A |
| 10 | $C_4H_9$ | 0 | 2 | -CH=CH- | $-NO_2$ | Cr198N>310 | $C_4H_9$-I.d3A |
| 11 | $C_4H_9$ | 0 | 2 | $-CH_2-CH_2-$ | -NCS | Cr153N269I | $C_4H_9$-I.d4C |
| 12 | $C_2H_5$ | 1 | 1 | $-COCH_2-$ | $-NO_2$ | Cr183N198I | $C_2H_5$-I.c1A |
| 13 | $C_3H_7$ | 1 | 1 | $-COCH_2-$ | $-NO_2$ | Cr175N217I | $C_3H_7$-I.c1A |
| 14 | $C_4H_9$ | 1 | 1 | $-COCH_2-$ | $-NO_2$ | Cr160N214I | $C_4H_9$-I.c1A |
| 15 | $C_6H_{13}$ | 1 | 1 | $-COCH_2-$ | $-NO_2$ | Cr152N219I | $C_6H_{13}$-I.c1 A |
| 16 | $C_7H_{15}$ | 1 | 1 | $-COCH_2-$ | $-NO_2$ | Cr136N196I | $C_7H_{15}$-I.c1A |
| 17 | $C_8H_{17}$ | 1 | 1 | $-COCH_2-$ | $-NO_2$ | Cr130N201I | $C_8H_{17}$-I.c1A |
| 18 | $C_{10}H_{21}$ | 1 | 1 | $-COCH_2-$ | $-NO_2$ | Cr132N192I | $C_{10}H_{21}$-I.c1A |
| 19 | $C_2H_5$ | 1 | 1 | $-CHOHCH_2-$ | $-NO_2$ | Cr128N195I | $C_2H_5$-I.c2A |
| 20 | $C_3H_7$ | 1 | 1 | $-CHOHCH_2-$ | $-NO_2$ | Cr123N213I | $C_3H_7$-I.c2A |
| 21 | $C_4H_9$ | 1 | 1 | $-CHOHCH_2-$ | $-NO_2$ | Cr117N215.5I | $C_4H_9$-I.c2A |
| 22 | $C_6H_{13}$ | 1 | 1 | $-CHOHCH_2-$ | $-NO_2$ | Cr107N192I | $C_6H_{13}$-I.c2A |
| 23 | $C_8H_{17}$ | 1 | 1 | $-CHOHCH_2-$ | $-NO_2$ | Cr94N195.5I | $C_8H_{17}$-I.c2A |
| 24 | $C_{10}H_{21}$ | 1 | 1 | $-CHOHCH_2-$ | $-NO_2$ | Cr91S160N189I | $C_{10}H_{21}$-I.c2A |
| 25 | $C_2H_5$ | 1 | 1 | -CH=CH- | $-NO_2$ | Cr178N>310 | $C_2H_5$-I.c3A |
| 26 | $C_3H_7$ | 1 | 1 | -CH=CH- | $-NO_2$ | Cr183N>320 | $C_3H_7$-I.c3A |
| 27 | $C_4H_9$ | 1 | 1 | -CH=CH- | $-NO_2$ | Cr172N>310 | $C_4H_9$-I.c3A |
| 28 | $C_6H_{13}$ | 1 | 1 | -CH=CH- | $-NO_2$ | Cr143N>310 | $C_6H_{13}$-I.c3A |
| 29 | $C_7H_{15}$ | 1 | 1 | -CH=CH- | $-NO_2$ | Cr140N>320 | $C_7H_{15}$-I.c3A |
| 30 | $C_8H_{17}$ | 1 | 1 | -CH=CH- | $-NO_2$ | Cr139N>310 | $C_8H_{17}$-I.c3A |
| 31 | $C_{10}H_{21}$ | 1 | 1 | -CH=CH- | $-NO_2$ | Cr134N>310 | $C_{10}H_{21}$-I.c3A |
| 32 | $C_2H_5$ | 1 | 1 | $-CH_2-CH_2-$ | $-NH_2$ | Cr180N209I | $C_2H_5$-I.c4B |
| 33 | $C_4H_9$ | 1 | 1 | $-CH_2-CH_2-$ | $-NH_2$ | Cr176N222I | $C_4H_9$-I.c4B |
| 34 | $C_6H_{13}$ | 1 | 1 | $-CH_2-CH_2-$ | $-NH_2$ | Cr169N214I | $C_6H_{13}$-I.c4B |
| 35 | $C_8H_{17}$ | 1 | 1 | $-CH_2-CH_2-$ | $-NH_2$ | Cr170N202I | $C_8H_{17}$-I.c4B |
| 36 | $C_{10}H_{21}$ | 1 | 1 | $-CH_2-CH_2-$ | $-NH_2$ | Cr164N192I | $C_{10}H_{21}$-I.c4B |
| 37 | $C_2H_5$ | 1 | 1 | $-CH_2-CH_2-$ | -NCS | Cr101N249I | $C_2H_5$-I.c4C |
| 38 | $C_3H_7$ | 1 | 1 | $-CH_2-CH_2-$ | -NCS | Cr122N266I | $C_3H_7$-I.c4C |
| 39 | $C_4H_9$ | 1 | 1 | $-CH_2-CH_2-$ | -NCS | Cr87N260I | $C_4H_9$-I.c4C |
| 40 | $C_6H_{13}$ | 1 | 1 | $-CH_2-CH_2-$ | -NCS | Cr76N248I | $C_6H_{13}$-I.c4C |
| 41 | $C_7H_{15}$ | 1 | 1 | $-CH_2-CH_2-$ | -NCS | Cr77N244I | $C_7H_{15}$-I.c4C |
| 42 | $C_8H_{17}$ | 1 | 1 | $-CH_2-CH_2-$ | -NCS | Cr60$S_A$75N235I | $C_8H_{17}$-I.c4C |
| 43 | $C_{10}H_{21}$ | 1 | 1 | $-CH_2-CH_2-$ | -NCS | Cr94$S_B$96N225I | $C_{10}H_{21}$-I.c4C |
| 44 | $C_2H_5$ | 1 | 1 | $-COCH_2-$ | -F | Cr158N164I | $C_2H_5$-I.c1D |
| 45 | $C_4H_9$ | 1 | 1 | $-COCH_2-$ | -F | Cr138N168I | $C4H_9$-I.c1D |
| 46 | $C_2H_5$ | 1 | 1 | -CH=CH- | -F | Cr152N302I | $C_2H_5$-I.c3D |
| 47 | $C_4H_9$ | 1 | 1 | -CH=CH- | -F | Cr164N310I | $C_4H_9$-I.c3D |
| 48 | $C_2H_5$ | 1 | 1 | $-CH_2-CH_2-$ | -F | Cr130N186 | $C_2H_5$-I.c4D |
| 49 | $C_4H_9$ | 1 | 1 | $-CH_2-CH_2-$ | -F | Cr134N201 | $C_4H_9$-I.c4D |
| 50 | $C_3H_7$ | 1 | 1 | -OCO- | -F | Cr128N288I | $C_3H_7$-I.c13D |
| 51 | $C_3H_7$ | 1 | 1 | -OCO- | -NCS | Cr155N300I | $C_3H_7$-I.c13C |
| 52 | $C_3H_7$ | 1 | 1 | -OCO- | $-NO_2$ | Cr149N318I | $C_3H_7$-I.c13A |

properties are revealed by the compounds with 4 to 8 carbon atoms in the $R^3$ alkyl chain (the range of the nematic phase is up to 180° and melthing enthalpy as low as 16 kJ/mole ). Table 2 gives the solubilities in wt.% of the compounds $C_4H_9$-I.c4C and $C_6H_{13}$-I.c4C in trans-4-(4-n-hexylcyclohexyl)benzeneisothiocyanate at various temperatures.

Table 2

| Compound | Temperature | | | | |
|---|---|---|---|---|---|
| | 30 °C | 20 °C | 10 °C | 0 °C | -10 °C |
| $C_4H_9$-I.c4C | 40.2 | 32.1 | 25.1 | 19.2 | 14.1 |
| $C_6H_{13}$-I.c4C | 42.3 | 30.6 | 21.7 | 14.5 | 9.2 |

The liquid crystal composition according to the invention is a mixture of at least two liquid crystal components of which at least one is a compound of the general formula I:

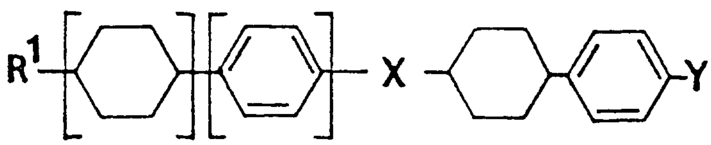

I.

wherein $R^1$ is a normal or branched alkyl group $H_{2n+1}C_n$- or a normal or branched alkoxy group $H_{2n+1}C_nO$-, n being an integer number from 1 to 12; X is a bridging group from the set: -COO-, -OCO-, $-CH_2CH_2-$, $-CHOCH_3CH_2-$; Y is a terminal group from the set: -NCS, the cyclohexane rings are trans-1,4-disubstituted ones and the benzene rings are 1,4-disubstituted ones, and besides one of them may contain sometimes additionally a fluorine atom in position 2 or 3; k and l are integer numbers from the set: 0, 1, 2 fulfilling the condition that k + l = 2 or 3; besides, if X denotes the -COO- or -OCO- group, then k + l also may assume the value of 1 ( k = 1 and l = 0 or k = 0 and l-1 ).

The liquid crystal admixtures according to the invention contains at least one component of the general formula I in a quantity exceeding 2 wt.% preferably 5 to 50 wt.% and if necessary also other non-liquid crystalline compounds such as dyes, solvents and optically active substances.

It has been ascertained that the mixtures containing the compounds of the general formula I have the required, advantageous for application properties, discussed at the beginning of the description, such as low viscosity and particulary low viscosity at low temperatures. The threshold voltage of the liquid crystal display may easily be varied in a wide range, but the temperature dependence being small in a wide range of temperatures. This facilitates the control of the electro-optical devices.

The compounds of the general formula I and their mixtures may be used, in principle, in any electrooptical device, it is preferred, however, that they be used outdoors where the temperature may vary in a wide range and high resistance to humidity and U.V. radiation is required. The devices utilizing these compounds may operate both in a direct control and multiplex control regime. The operation of the liquid crystal display filled with the liquid crystal mixture accordnig to the invention may be based on any electrooptical effect though particularly preferred are those taking advantage of the twisted nematic or guest-host effect. The dichroic dyes necessary in the case of the latter effect-such as anthraquinone ones-reveal excellent solubility in the mixtures according to the invention, and these mixtures give high contrast. The mixture according to the invention raises significantly the performance of the electrooptical devices in which it is used whose construction is generally known.

The mixtures according to the invention may include alongside with one or several compounds of formula I additionally other liquid crystal compounds as, e.g. compounds selected from such classes of compounds as biphenyls, phenylcyclohexanes, phenylcyclohexylethanes, phenyldioxanes, phenylpyrimidines, phenyl esters of benzoic or cyclohexanecarboxylic acids, Schiff's bases, azoxy compounds, cyclohexylbiphenyls, diphenylpyrimidines, biphenylates.

Among the enumerated classes of compounds the compounds of the following formulae are preferred:

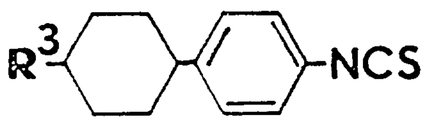

XVIII.

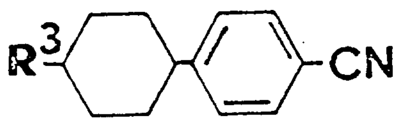

XIX.

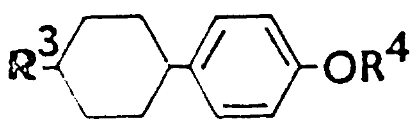

XX.

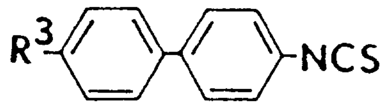

XXI.

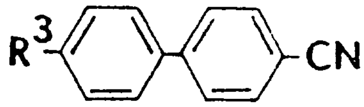

XXII.

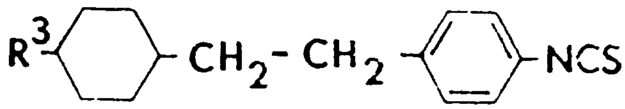

XXIII.

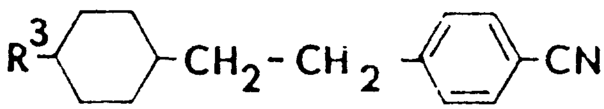

XXIV.

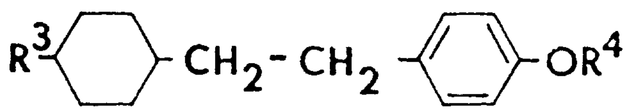

XXV.

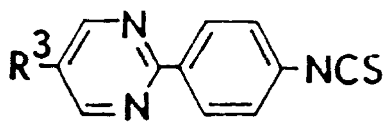

XXVI.

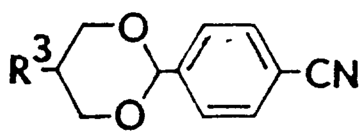

XXVII.

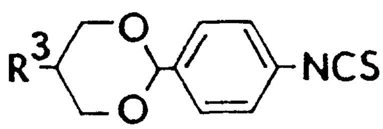

XXVIII.

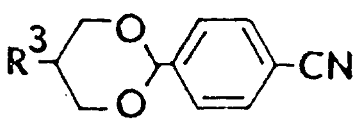

XXIX.

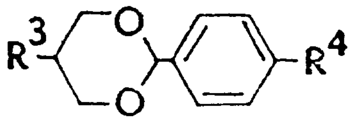

XXX.

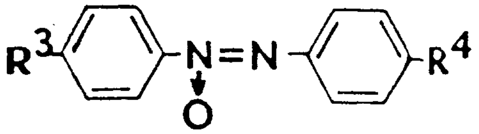

XXXI.

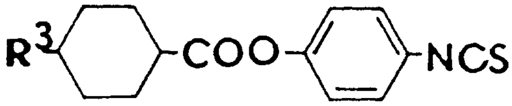

XXXII.

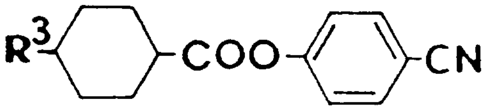

XXXIII.

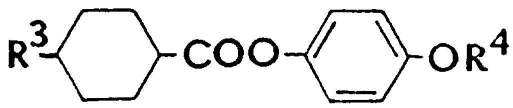

XXXIV.

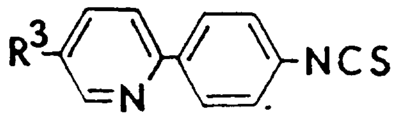

XXXV.

where $R^3$ is an alkyl group including from 1 to 8 carbon atoms, and $R^4$ is an alkyl group containing from 1 to 5 carbon atoms. Among the compounds with formulae from XVIII to XXXV the most preferred components of the mixtures are those of formualae XVIII, XIX, XXI, XXIII, XXIV, XXVI, XXVII, XXXV. Especially compound XVIII is suitable for producing base mixtures. As regards compounds XVIII, XXI, XXIII, XXVIII, XXXV, it is advantageous to combine them with compounds of the general formula I.cC which have alkyl groups with 2 to 8 carbon atoms. Especially recommended is the compound of formula $C_4H_9$-I.c4C which shows the phase transitions Cr87N260I, and $C_6H_{13}$-I.c4C which shows the phase transitions Cr74N247.5I.

The compound or compounds of formula I and compounds of formulae from XIV to XXXI yield mixtures of advantageous properties if the former constitute 5 to 50 wt.%. Compounds of formulae XXI, XXVI, XXVIII have a smectic A or E phase, so it is not recommended that they be used alone with the compound or compounds of formula I but together with the compound of formula XVIII or XXIII, and their proportion in the mixture should not exceed 30 wt.%.

The compounds of formula I reveal high clearing points, so non-liquidcrystalline compounds or a compound revealing mesogenic properties at low temperatures may also be added to the mixtures containing the former. Among the enumerated compounds those with formula XXXVI and XXXVII are advantageous compounds of the mixtures.

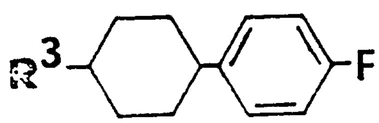

XXXVI.

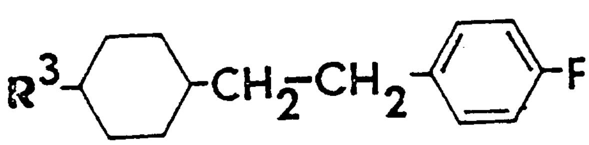

XXXVII.

Examples are given below characterizing the process of preparing the liquid crystal compounds and of the necessary substrates as well as the application of these compounds in liquid crystal mixtures. The examples are given to illustrate the invention rather than to limit its range. The temperatures are in all instances given in °C.

Example 1

trans-4-(4-Nitrophenyl)cyclohexanecarboxylic acid (formula II.3A)

(a)

A mixture of 204 g trans-4-phenylcyclohexanecarboxylic acid, 300 $cm^3$ $CCl_4$, 96 g methanol and 5 $cm^3$ conc. $H_2SO_4$ is prepared and refluxed for 12 hours with protection from humidity. Next the contents are diluted with water and the phases are separated. The aqueous phase is extracted with $CCl_4$, and the combined organic phases are washed with water, 5% $NaHCO_3$ and dried with $MgSO_4$. The drying agent is filtered off and the remainder after distilling off the solvent is distilled under vacuum and the 113-115°/0.1mmHg fraction being colleted. The yield of the methyl ester of trans-4-phenylcyclohexanecarboxylic acid is 198 g (91%), m.p. 34-35°.

(b)

A mixture of 172 g methyl ester of trans-4-phenylcyclohexanecarboxylic acid and 70$cm^3$ nitrobenzene is cooled to 5° when a mixture of 150 $cm^3$ of 96% $H_2SO_4$ and 88 $cm^3$ of 65% $HNO_3$ is added dropwise with vigorous stirring, the temperature being kept in the range +5° - +10°. After adding the nitrating acids stirring is continued for 3 hours and the contents are poured onto ice. Then 400 $cm^3$ of $CCl_4$ are added with vigorous agitation. From the obtained emulsion crystals precipitate. They are filtered, washed with water and dried. Next the organic layer of the filtrate is separated and it is washed with water, 5% $NaHCO_3$, dried with $MgSO_4$ and filtred. Carbon tetrachloride and nitrobenzene are distilled off under vacuum and the residue is crystallized from methanol. Both fraction of crystals, 162 g, are recrystallized from 2:1 mixture of hexane and benzene. The yield of the methyl ester of trans-4-(4-nitrophenyl)cyclohexanecarboxylic acid of m.p. 127-128.5° and purity 97% is 133 g (64%).

(c)

40 g of the methyl ester of trans-4-(4-nitrophenyl)cyclohexanecarboxylic acid and a solution of 12 g of NaOH in 250 $cm^3$ of water are refluxed until a clear solution is obtained. The solution is then cooled and acidified with hydrochloric acid. The precipitate is filtered, washed with water and dried. A yield of 37 g of trans-4-(4-nitrophenyl)cyclohexanecarboxylic acid (about 100%) is obtained with the phase transitions Cr182-165N240I with decomposition.

Example 2

trans-4-(4-Nitrophenyl)cyclohexylacetic acid (formula II.1A)

(a)

A mixture of 152 g of trans-4-acetylcyclohexylbenzene, 37.5 g of sulphur and 105 $cm^3$ of morpholine is refluxed for 10 hours. It is then poured into 750$cm^3$ of rectified ethanol and cooled overnight at 0°. Next the crystals are filtered, a yield of 178 g of thiomorpholide of m.p. 135-140° being obtained. After drying thiomorpholide is hydrolyzed in a mixture of 320 $cm^3$ of acetic acid, 47.5 $cm^3$ conc. $H_2SO_4$ and 74 $cm^3$ of water. Upon refluxing for 4 h the evolution of hydrogen sulphide practically ceases. A dark tarry residue remains at the bottom of the flask. The upper layer is separated by pouring it into 1500 $cm^3$ of cold water. The precipitated trans-4-phenylcyclohexylacetic acid is filtered, washed with water and dissolved in a solution of 30g of NaOH in 1200$cm^3$ of water. The undissolved substances are filtered off, the filtrate is acidified, upon which trans-4-phenylcyclohexylacetic acid precipitates. The precipitate is filtered and dried. Crude trans-4-phenylcyclohexylacetic acid, 152 g, 350 $cm^3$ of $CCl_4$, 66 $cm^3$ of methanol and 3 $cm^3$ conc. $H_2SO_4$ are refluxed for 12 h., upon which the mixture is diluted with water and the phases separated. The organic phase is washed with water, 5% $NaHCO_3$, water again, and after drying with $MgSO_4$ the solvent is

distilled off. The residual oil is distilled under vacuum, the 132-138°/0.3 mmHg fraction being collected. A yield of 103g (55%) of the methyl ester of trans-4-phenylcyclohexylacetic acid is obtained.

(b)

90g of methyl ester of trans-4-phenylcyclohexylacetic acid are cooled to 10° when the nitrating mixture of 41cm$^3$ conc. $HNO_3$ and 51 cm$^3$ conc. $H_2SO_4$ is added dropwise. After stirring for 4h the contents are poured onto ice and the product is extracted with $CCl_4$. The extracts are combined, washed with water and 5% $NaHCO_3$, and dried with $MgSO_4$. After distilling off $CCl_4$ 125 g of yellow oil are obtained which is three times crystallized from the 5:1 hexanebenzene mixture. A yield of 41g (38%) of methyl ester of trans-4-(4-nitrophenyl)cyclohexylacetic acid of m.p. 65-66° is obtained.

(c)

A 41g portion of the methyl ester of trans-4-(4-nitrophenyl)cyclohexylacetic acid is hydrolized analogously as in Example 1. The yield of the crude trans-4-(nitrophenyl)cyclohexylacetic acid is 38g (98%). After crystallization from 3:1 benzene-hexane mixture it has m.p. 135-137°.

Example 3

trans-4-(4-Fluorophenyl)cyclohexylmethanol (II.5D)

(a)

66 g of methyl ester of trans-4-(4-nitrophenyl)cyclohexanecarboxylic acid (obtained in Example 1) are dissolved in 600cm$^3$ of ethyl acetate and 2 g of 5% Pd on active carbon are added. The contents are reduced with gaseous hydrogen under normal pressure with vigorous stirring, until the absorption of hydrogen ceases. Next the catalyst is filtred off and the filtrate is concentrated in a vacuum evaporator. A yield of 65 g of the aminoester of m.p. 54-64° are obtained.After crystalization from methanol, the methyl ester of trans-4-(4-aminophenyl)cyclohexanecarboxylic acid of m.p. 62-64° is obtained.

(b)

The methyl ester of trans-4-(4-aminophenyl)cyclohexanecarboxylic acid is dissolved in 200 cm$^3$ of ethyl alcohol and the solution is added dropwise with vigorous mixing into 1500 cm$^3$ of water. The obtained fine crystalline amine is filtered, washed with water and suspended in a mixture of 200 cm$^3$ of water and 78.5 cm$^3$ of conc. HCl. The obtained suspension is cooled to 0-5° and diazotized by adding dropwise 20 g $NaNO_2$ in 75 cm$^3$ of water. The solution of the diazonium salt is filtered and then 38 g of $NaBF_4$ in 75 cm$^3$ of water are added to the filtrate. Diazonium tetrafluoroborate is filtered, washed with cold water, methanol, and ether. A yield of 64 g the dry diazonium salt melting et 107-110° with decomposition is obtained.

(c)

The diazonium salt obtained in step (b) is suspended in 1200 cm$^3$ of gasoline (70-110° fraction) and heated to distil slowly off the volatile components. The decomposition of diazonium tetrafluoroborate starts at about 80°. After the evolution of nitrogen and boron fluoride ceases, the mixture is filtered, the filtrate is washed with water and 5% $NaHCO_3$, again with water and dried with $MgSO_4$. Next the drying agent is filtered off and gasoline solution is concentrated. The remainder is distilled under vacuum (oil pump) the 120-125°/0.08 mmHg fraction being collected. A yield of 34.5 g (57.5%) of the methyl ester of trans-4-(4-fluorophenyl)-cyclohexanecarboxylic acid in the form of a colorless low-melting solid of m.p. 48-54° is obtained.

(d)

A solution of 76 g of the methyl ester of trans-4-(4-fluorophenyl)cyclohexanecarboxylic acid in 200 cm$^3$ of ether is added dropwise to a mixture of 400 cm$^3$ of diethyl ether and 10 g of $LiBH_4$ and the obtained mixture is heated for 3 h under reflux. After cooling the excess hydride is decomposed by adding dropwise 150cm$^3$ of water acidified with $H_2SO_4$. The phases are then separated, and the water phase is extracted

with ether. The combined other extracts are washed with brine and dried with $MgSO_4$. Ether is distilled off and the residue is crystallized from 250cm$^3$ of hexane. A yield of 58g (87%) of trans-4-(4-fluorophenyl)-cyclohexylmethanolof m.p. 75-77° is obtained.

Example 4

Trans-4-(4-fluorophenyl)cyclohexylacetic acid (II.1D)

(a)

To 58g of trans-4-(4-fluorophenyl)cyclohexylmethanol (obtained in step (d) of Example 3) 27.3 cm$^3$ of pyridine and 100cm$^3$ of benzene are added. Then 95 g of thionyl chloride are slowly added dropwise without cooling and then the contents are heated under reflux for 4 h. The mixture is next cooled, poured onto water with ice and the phases are separated. The water phase is extracted with benzene. The combined organic phases are washed with water, 5% $NaHCO^3$ and again with water, and dried with $MgSO_4$. The drying agent is then filtered off, and the filtrate is concentrated and distilled under vacuum, the 106-109°/0.08 mmHg fraction being coolected.A yield of 58 g (91%) of trans-4-(4-fluorophenyl)cyclohexylmethyl chloride is obtained of m.p. 68-70°.

(b)

A mixture of 58 g trans-4-(4-fluorophenyl)cyclohexylmethylchloride, 120 cm$^3$ triethylene glycol, 16.7g NaCN is heated with stirring to 104° at which temperature it is kept for 2h. Next the contents are poured into 1000cm$^3$ of water. The precipitate is filtered off and the filtrate is extracted with chloroform. The precipitate is then dissolved in chloroform and combined with the extract, washed with water, 50% $H_2SO_4$, again with water and dried with $MgSO_4$. After filtration and concentration a yield of 55 g (99%) of the crude trans-4-(4-fluorophenyl)cyclohexylmethyl cyanide is obtained, which after crystallization from hexane melts at 88°.

(c)

The cyanide obtained in step (b) is hydrolized to the acid by heating for 24 h in a mixture of 75cm$^3$ of acetic acid, 60 cm$^3$ of water end 48 cm$^3$ of conc. $H_2SO_4$. The reaction mixture is poured into 1500 cm$^3$ of water and filtered. The crude acid is dissolved in a solution of 40 g of NaOH in 500 cm$^3$ of water. The solution is filtered through a layer of active carbon and acidified. The precipitated trans-4-(4-fluorophenyl)-cyclohexylacetic acid is filtered, dried and crystallized from a 2:1 hexane-benzene mixture. A yield of 42g (70%) of trans-4-(4-fluorophenyl)-cyclohexyl-acetic acid of m.p. 128-130° is obtained.

Example 5

trans-4-(4-Nitrophenyl)cyclohexylmethanol (II.5A)

(a)

37.3 g of trans-4-(4-nitrophenyl)cyclohexanecarboxylic acid are heated with 47 g of oxalyl chloride.After the excess of oxalyl chloride is distilled off, the crude acid chloride is obtained.

(b)

The trans-4-(4-nitrophenyl)cyclohexanecarboxylic acid chloride (II.4A) obtained in step (a) is dissolved in 80 cm$^3$ of anhydrous diglyme and the solution is added drowise to a flask with a supension of 9,5 g of $NaBH_4$ in 200 cm$^3$ diglyme the temperature being kept at 35-40°, The contents of the flask are agitated for 3h and poured into 1000 cm$^3$ of water acidified with 50 cm$^3$ of conc. HCl. The precipitate is filtered off, and the filtrate is extracted with ether. The precipitate is dissolved in ether and combined with the extract, washed with brine, dried with $MgSO_4$, filtered and concentrated. A yield of 34 g (95%) of the crude compound II.5A is obtained which after crystallization from a 1:2 hexane-benzene mixture has a m.p. of 115°.

Example 6

trans-4-(4-Nitrophenyl)cyclohexylmethyl chloride (II.6A)

The crude compound II.5A obtained in Example 5 is converted to the chloride by heating with thionyl chloride and pyridine analogously as in Example 4. After concentration the solid residue is crystallized from acetone.The yield is 25.7g(68%) of II.6A of m.p. 73-74°.

Example 7

4-[trans-4-(4-Nitrophenyl)cyclohexylacetyl]-4'-n-butybiphenyl ($C_4H_9$-I.d1A)

(a)

A mixture of 5.26g trans-4-(4-nitrophenyl)-cyclohexylacetic acid and 5.2 $cm^3$ of oxalyl chloride is prepared, and it is heated under reflux for 2 h.Then excess of oxalyl chloride is distilled off under vacuum, and the obtained crude trans-4-(4-nitrophenyl)cyclohexylacetyl chloride is used in the next step.

(b)

3.06 g of anhydrous aluminum chloride are added to 150 $cm^3$ of methylene chloride. The mixture is cooled to 5° when trans-4-(4-nitrophenyl)cyclohexylacetyl chloride obtained in step (a) and dissolved in 150 $cm^3$ of methylene chloride is added dropwise with stirring. The mixture is vigorously stirred For 30 min upon which 5.25 g of 4-n-butylbiphenyl is slowly added dropwise keeping the temperature in the range of 0-5°. After continued mixing for a further 3h the reaction mixture is poured into 50 $cm^3$ of 5% HCl with ice. The phases are separated, and the aqueous phase is extracted with 50 $cm^3$ of methylene chloride. The organic phases are combined, washed with 5% HCl, water to a neutral reaction, and dried with $MgSO_4$. The solvent is distilled off and the residue is crystallized from a 2:1 ethanol-acetone mixture. The yield of the $C_4H_9$-I.d1A compound is 4.1 g (45%) and its phase transition points are: Cr152N219I.

The compounds: $C_5H_{11}$-I.d1A, $C_2H_5$-I.c1A, $C_3H_7$-I.c1A, $C_4H_9$-I.c1A, $C_5H_{11}$-I.c1A, $C_6H_{13}$-I.c1A, $C_7H_{15}$-I.c1A, $C_8H_{17}$-I.c1A, $C_9H_{19}$-I.c1A are obtained in a similar manner; their phase transition points are listed in Table 1.

Example 8

1-(4'-n-Butylbiphenylyl-4)-2-[trans-4-(4-nitrophenyl)cyclohexyl]ethanol ($C_4H_9$-I.d2A)

4.10 g of $C_4H_9$-I.d1A dissolved in mixture of 150 $cm^3$ of anhydrous ethanol and 100 $cm^3$ of dimethoxyethane. To the agitated solution 1g of $NaBH_4$ is added in two portions at 1h interval and the reaction mixture is left to stand for 24 h. Next the solvent is distilled off under vacuum, the residue is diluted with water, acidified with dilute hydrochloric acid and the precipitate is filtered. The crude product is crystallized from a 3:1 ethanol-acetone mixture, The yield of compound of $C_4H_9$-I.d2A is 3.55 g (86.3%) and its phase transition points are: Cr135N226I.

The compounds: $C_2H_5$-I.d2A, $C_3H_7$-I.d2A, $C_2H_5$-I.c2A, $C_3H_7$-I.c2A, $C_4H_9$-I.c2A, $C_5H_{11}$-I.c2A, $C_6H_{13}$-I.c2A, $C_7H_{15}$-I.c2A, $C_8H_{17}$-I.c2A, $C_9H_{19}$-I.c2A $C_{10}H_{21}$-I.c2A are obtained in the similar manner.

Example 9

1-(4'-n-Butylbiphenylyl-4)-2-[trans-4-(4-nitrophenyl)cyclohexyl]ethene ($C_4H_9$-I.d3A)

3.55 g of compound $C_4H_9$-I.d2A are dissolved in 130 $cm^3$ of toluene, a catalytic quantity of p-toluenesulfonic acid is added and the reaction mixture is refluxed, the evolving water being removed by applying an azeotropic trap. Next the mixture is cooled, washed with a 5% $NaHCO_3$ solution, water and dried with $MgSO_4$. After Filtration toluene is distilled off and the solid residue is crystallized from a 3:1 acetone - THF mixture. The yield of compound $C_4H_9$-I.d3A is 2.A5 g (83.5%) and its phase transition points are: Cr158N310>I (above 310° decomposition of the compound is observed).

The compounds: $C_2H_5$-I.c3A, $C_3H_7$-I.c3A, $C_4H_9$-I.c3A, $C_6H_{13}$-I.c3A, $C_7H_{15}$-I.c3A, $C_8H_{17}$-I.c3A, $C_9H_{19}$-I.c3A, $C_{10}H_{21}$-I.c3A are obtained in a similar manner.

Example 10

1-(4-n-Butylbiphenylyl-4)-2-[trans-4-(4-isotiocyanatophenyl)-cyclohexyl]ethane ($C_4H_9$-I.d4C)

To 2.85 g of $C_4H_9$-I.d3A 150 $cm^3$ of ethyl acetate and 300 mg of 5% Pd on active carbon are added. The mixture in the flask is connected to a gasometer and saturated with hydrogen until the absorption of the latter ceases (about 2 h). Next the mixture is heated to the boil and at that temperature the catalyst is filtered off. The filtrate is concentrated to complete evaporation of the solvent, the yield of dry 1-(4'-n-butylbiphenylyl-4)-2-[trans-4-(4-aminophenyl)cyclohexyl]ethane ($C_4H_9$ I.d4B) being 2.60 g. The crude product is dissolved in 25 $cm^3$ of chloroform and added dropwise with vigorous stirring at 5-10° to a mixture of 0.38 g of $CaCO_3$ and 0.62 $cm^3$ of thiophosgene in 10 $cm^3$ of chloroform and 10 $cm^3$ of water. Stirring is continued for a further 1h at room temperature. Next 2.5 $cm^3$ of 1N hydrochloric acid are added and the phases are separated. The organic phase is washed with water, dried with $MgSO_4$, filtered and the solvent distilled off. The dry residue is dissolved in benzene and filtered through a columm with silica gel in order to remove the color. The filtrate is concentrated by evaporation and the dry residue is crystallized from a 1:1:1 $CHCl_3$-ether-methanol mixture. The yield of the obtained $C_4H_9$-I.d4C is 1.76 g (63%) and its phase transition points are: Cr153N269I.

The compounds: $C_2H_5$-I.c4C, $C_3H_7$-I.c4C, $C_4H_9$-I.c4C, $C_6H_{13}$-I.c4C, $C_5H_{11}$-I.c4C, $C_7H_{15}$-I.c4C, $C_8H_{17}$-I.c4C, $C_9H_{19}$-I.c4C, $C_{10}H_{21}$-I.c3C, $C_{12}H_{25}$-I.c4C are obtained in a similar manner.

Example 11

4'-n-Butylphenyl ester of trans-4-(4-nitrophenyl)cyclohexane carboxylic acid ($C_4H_9$-I.b13A)

(a)

To 7.50 g of trans-4-(4-nitrophenyl)cyclohexanecarboxylic acid 7.5 $cm^3$ of oxalyl chloride are added and the mixture is refluxed for 2 h. Excess of oxalyl chloride is distilled off under vacuum, and the obtained crude trans-4-(4-nitrophenyl)cyclohexanecarboxylic acid chloride is used in the next step.

(b)

To 50 $cm^3$ of benzene 4.50 g of 4-n-butylphenol and 4.75 g of anhydrous pyridine are added. trans-4-(4-Nitrophenyl)cyclohexanecarboxylic acid chloride obtained in step (a) is dissolved in 20 $cm^3$ of benzene and added dropwise to the stirred solution of phenol in benzene, the temperature being kept at 15-20°. The reaction mixture is then refluxed for 1 h, cooled, washed with a 5% HCl solution, and dried with $MgSO_4$. After filtering, the filtrate is concentrated, and the dry residue is crystallized twice from ethanol. The yield of compound $C_4H_9$-I.b13A is 6.80 g (59%) and its phase transition points are: Cr114N150I. The compounds: $C_4H_9$-I.a13A, $C_3H_7$-I.a13A, $C_5H_{11}$-I.a13A are obtained in a similar manner.

Example 12

4-n-Butylphenyl ester of trans-4-(4-isothiocyanatophenyl)cyclohexanecarboxylic acid ($C_4H_9$-I.b13C)

(a)

4.50g of compound $C_4H_9$-I.b13A are reduced with gaseous hydrogen in analogous manner as in Example 10 in the presence of 50 $cm^3$ of ethyl acetate and 100 mg of 5% Pd on active carbon. The yield of the obtained ester $C_4H_9$-I.b13B is 4.0 g and its phase transition points are Cr107N124I.

(b)

The compound $C_4H_9$-I.b13B obtained in step (a) is dissolved in 30 $cm^3$ of chloroform and added dropwise,with vigorous stirring, to a mixture prepared of 2.0 g $CaCO_3$, 1.13 $cm^3$ of thiophosgene in 30 $cm^3$ of chloroform and 10 $cm^3$ water. The temperature being kept at 5-10°. After further stirring for 1h, 10$cm^3$ of 1N hydrochloric acid are added and the phases are separated. The organic phase is washed with water and dried with $MgSO_4$. After filtration, chloroform is distilled off under vacuum and the residue is crystallized twice from a 3:1 methanol-ether mixture. The yield of compound $C_4H_9$-I.b13C is 2,55 g (55%) and its phase

transition points are:Cr 107 N 189 I. The compounds: $C_4H_9$-I.a13C, $C_3H_7$-I.c13C, $C_5H_{11}$-I.a13C are obtained in a similar manner.

Example 13

trans-4-(4-Fluorphenyl)cyclohexylacetyl-4-[trans-4-(ethyl cyclohexyl]benzene ($C_2H_5$-I.c1D)

(a)

From 9.4g of the trans-4-(4-fluorophenyl)cyclohexylacetic acid and 8 $cm^3$ of oxalyl chloride, the acid chloride is prepared in an analogous manner as in example 7a

(b)

8.0g of anhydrous aluminum chloride are added to 50 $cm^3$ of methylene chloride and to this mixture, the trans-4-(4-fluorophenyl)cyclohexylacetyl chloride prepared in step (a) and dissolved in 20 $cm^3$ methylene chloride, is added dropwise at 5°, with vigorus stirring. Stirring is continued for a further 20 min after which 7.55g of trans-(4-ethylcyclohexyl)benzene are added dropwise, the temperature being kept at 0-5°. Stirring is continued for 4h and then the mixture is poured into 100$cm^3$ of 5% HCl with ice, the phases are separated, the aqueous phase being extracted with 50$cm^3$ of methylene chloride. The combined organic phases are washed with 5% HCl,water to neutral reaction, and dried with $MgSO_4$. The solvent is distilled off and the residue is crystallized first from a 2:1 ethanol-acetone mixture, then from heptane and finally again from the ethanol-acetone mixture. The yield of compound $C_5H_{11}$-I.c1D is 7.7g (47.5%) and its phase transition points are: Cr158N164I.

The compounds: $C_4H_9$-I.c1D, $C_5H_{11}$-I.c1D, $C_6H_{13}$-I.c1D are obtained in a similar manner.

Example 14

1-[4-(trans-4-ethylcyclohexyl)phenyl]-2-[trans-4-(4-fluorophenyl)cyclohexy]ethanol ($C_2H_5$-I.c2D)

7.7g of $C_2H_5$-I.c1D are dissolved in a mixture of 100 $cm^3$ of THF and 100 $cm^3$ of anhydrous ethanol. To the mixture 2g of $NaBH_4$ are added in two portions in a several hour interval and the reaction mixture is heated to 40° and left to stand for 24h. Next the solvent is distilled off under vacuum, and the residue is diluted with water, acidified with dilute HCl and the precipitate filtered. After crystallized from hexane the yield of compound $C_2H_5$-I.c2D is 5.2g (67%), its m.p. is 128-130°. Compound $C_4H_9$-I.c2D is obtained in an analogous manner.

Example 15

1-[4-(trans-4-ethylcyclohexyl)phenyl]-2-[trans-4-(4-fluorophenyl)cyclohexyl]ethene ($C_2H_5$-I.c3D)

5.2g of compound $C_2H_5$-I.c2D are dissolved in 150 $cm^3$ of toluene, 100mg of p-toluanesulfonic acid are added and the mixture is heated under reflux, the evolving water being removed in an azeotropic trap. Next the mixture is diluted with toluene to dissolved the crystallizing product, washed with a 5% $NaHCO_3$ solution and water, then dried with $MgSO_4$. After filtration, the toluene is distilled off from the filtrate and the residue is crystallized from a 2:1 hexane-benzene mixture. The yield of compound $C_2H_5$-I.c3D is 4.8g, its phase transition points are: Cr164N310I. Compound $C_4H_9$-I.c3D is obtained in an analogous manner.

Example 16

1-[4-(trans-4-ethylcyclohexyl)phenyl]-2-[trans-4-(4-fluorophenyl)cyclohexyl]ethane ($C_2H_5$-I.c4D)

4.8g of compound $C_2H_5$-I.c3D are added to 150 $cm^3$ of ethyl acetate and in presence of 500 mg of Pd on active carbon the mixture is saturated with hydrogen until the absorption of the latter ceases. Then the catalyst is filtered from the hot solution, the filtrate is concentrated, and the solid residue is crystallized twice from hexane. The yield of compound $C_2H_5$-I.c4D is 3.04 g (63%) and its phase transition points are: Cr120N186I. The compound $C_4H_9$-I.c4D is obtained in a similar manner.

Example 17

4-Butylphenyl ester of trans-4-(4-fluorophenyl)cyclohexanecarboxylic acid ($C_4H_9$-I.b13D)

(a)

The acid chloride is prepared from 3.85g of trans-4-(4-fluorophenyl)cyclohexanecarboxylic acid and 3.5 $cm^3$ of oxalyl chloride analogously as in Example 11.

(b)

The solution chloride of trans-4-(4-fluorophenyl)cyclohexanecarboxylic acid, obtained in step (a) , in 15 $cm^3$ of benzene is added dropwise at 15-20° to a mixture of 30 $cm^3$ of benzene, 2.55g 4-n-butylphenyl and 2.7 $cm^3$ anhydrous pyridine. Next the mixture is refluxed for 1h, cooled and washed with 5% HCl, water, dried with $MgSO_4$ and filtered. The filtrate is concentrated and the residue is crystallized from, ethanol and hexane. The yield of compound $C_4H_9$-I.b13D is 3.80g (61%) and its phase transition points are Cr94N116I. The compounds $C_5H_{11}$-I.a13D and $C_3H_7$-I.c13D are obtained in an analogous manner.

Example 18

Mixture A is prepared of the following composition in wt.%:

| | |
|---|---|
| 4-(trans-4-n-propylcyclohexyl)-1-isothiocyanatobenzene | 40 |
| 4-(trans-4-n-hexylcyclohexyl)-1-isothiocyanatobenzene | 42 |
| 4-(trans-4-n-octylcyclohexyl)-1-isothiocyanatobenzene | 18 |

The mixture has the following parameters:

| | | | | |
|---|---|---|---|---|
| 1. Nematic phase range: from below -30° to 41.5° | -20° | 0° | 20° | 40° |
| 2. Bulk viscosity [mPa.s] | 50 | 21.5 | 11 | 7.2 |
| 3. Threshold voltage $V_{10}$, [V] | -1.9 | 1.7 | 1.5 | 1.1 |

* When supercooled, m.p (-8°).

Mixture B is prepared of the following composition in wt.%:

| | |
|---|---|
| Mixture A | 85 |
| Compound $C_6H_{13}$-I.c4C | 15 |

The mixture has the following parameters:

| | | | | |
|---|---|---|---|---|
| 1. Nematic phase range: from below -35° to 73° | -20° | 0° | 20° | 40° |
| 2. Bulk viscosity [mPa.s] | 51 | 24 | 12.7 | 7.2 |
| 3. Threshold voltage $V_{10}$, [V] | 2.0 | 1.9 | 1.75 | 1.65 |

If the mixture is composed of 75 wt.% of A and 25 wt.% of compound $C_6H_{13}$-I.c4C, than an increase of the clearing temperature to 93° is obtained, as well as very good mutual solubility of the mixture components at room temperature. If 10 wt.% of 2-(4-cyanophenyl)-5-(4-n-butylphenyl)pyrimidine or 10 wt.% of 4-n-pentyl-4'-cyanoterphenyl or 10 wt.% of 4-pentylphenyl 4-(trans-4-pentylcyclohexyl)benzoate are added to Mixture A, then dissolving of the components takes place only after heating; on cooling a solid phase precipitates from the mixture.

Example 19

The mixture of the following composition in wt.%:

| | |
|---|---|
| 4-(trans-4-n-propylcyclohexyl)-1-isothiocyanatobenzene | 20.35 |
| 4-(trans-4-n-hexylcyclohexyl)-1-isothiocyanatobenzene | 18.46 |
| 4-n-pentyl-4'-isothiocyanatobiphenyl | 12.92 |
| 4-n-heptyl-4'-isothiocyanatobiphenyl | 13.53 |
| 1-(4'-n-hexylbiphenylyl-4)-2-(4-isothiocyanatophenyl)ethane | 9.23 |
| Compound $C_6H_{13}$-I.c4C | 16.00 |
| cholesteryl pelargonate | 0.28 |

has the parameters:

| | |
|---|---|
| 1. Nematic phase range: | from below -30° to 78° |
| 2. Bulk viscosity at 20° [mPa.s] | 19.5 |
| 3. Threshold voltage at 20° [V] | 2.2 |

Example 20

The mixture of the following composition in wt.%:

| | |
|---|---|
| 4-(trans-4-n-propylcyclohexyl)-1-isothiocyanatobenzene | 29.60 |
| 4-(trans-4-n-hexylcyclohexyl)-1-isothiocyanatobenzene | 31.08 |
| 4-(trans-4-n-octylcyclohexyl)-1-isothiocyanatobenzene | 18.32 |
| Compound $C_5H_{11}$-I.a13C | 12.00 |
| Compound $C_4H_9$-I.b13C | 8.00 |
| Compound $C_3H_7$-I.c13C | 6.00 |

has the clearing point at 86°,
and its bulk viscosity at 20° is 20.0 mPa s

Example 21

The mixture of the following composition in wt.%:

| | |
|---|---|
| 4-(trans-4-n-propylcyclohexy)-1-isothiocyanatobenzene | 23.46 |
| 4-(trans-4-n-butylcyclohexyl)-1-isothiocyanatobenzene | 12.95 |
| 4-(trans-4-n-hexylcyclohexyl)-1-isothiocyanatobenzene | 21.50 |
| 4-(trans-4-n-octylcyclohexyl)-1-isothiocyanatobenzene | 9.23 |
| 1-[4-(trans-4-n-butylcyclohexyl)phenyl]-2-(4-isothiocyanatophenyl)ethane | 5.44 |
| 1-[4-(trans-4-n-hexylcyclohexyl)phenyl]-2-(4-isothiocyanatophenyl)ethane | 5.44 |
| Compound $C_4H_9$-I.c4C | 10.90 |
| Compound $C_6H_{13}$-I.c4C | 10.90 |
| Optically active 1-[4'-(2-methylbutyl)biphenylylyl-4]-2-(4-isothiocyanatophenyl)ethane | 0.18 |

has the following parameters:

1. Nematic phase range: from below -40° to 97°

| | -20° | 0° | 20° | 40° | 60° |
|---|---|---|---|---|---|
| 2. Bulk viscosity [mPa·s] | 109 | 41 | 17.5 | 8.7 | 5 |
| 3. Threshold voltage $V_{10}$, [V] | 2.1 | 2.0 | 1.9 | 1.8 | 1.7 |

4. Temperature dependence of $V_{10}$ in the range from -20° to 60°

$$r_T = \frac{\sqrt{\phantom{x}}(V_1 - V_2)\ 100\%}{(V_1 + V_2)\ (T_1 - T_2}\qquad -0.26$$

5. Optical anisotropy at 20°, $\Delta n$ 0.16

6. Dielectric anisotropy at 20°, $\Delta\varepsilon = \varepsilon_{\parallel} - \varepsilon_{\perp}$ +7

## Claims

**1.** A cyclohexylbenzene derivative of the general formula

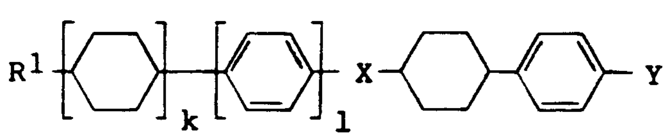

(I)

wherein $R^1$ is a normal or branched alkyl group $H_{2n+1}C_n$-or normal or branched alkoxy group $H_{2n+1}C_nO$-, n being an integer from 1 to 12; X is a bridging group $-CHOHCH_2-$, $-CH_2CH_2-$ or $-CHOCH_3CH_2$; -COO-, -OCO-; Y is a terminal group $-NO_2$, $-NH_2$ or -NCS; the cyclohexane rings are trans-1,4-disubstituted ones and the benzene rings are 1,4-disubstituted, in which the H-atom in 3- or 2-position can be replaced by F-atom; k and l are integers 0, 1 or 2, under the condition that k+1=2 or 3, besides if X denotes a -COO- or -OCO-group, k+1 also may be 1.

**2.** The compound having the formula

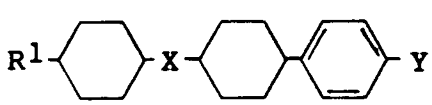

(Ia)

wherein $R^1$ is a normal or branched alkyl chain having 1 to 12 carbon atoms; X is a bridging group -COO- or -OCO-, Y is a terminal group $-NO_2$ or -NCS.

**3.** The compound having the formula

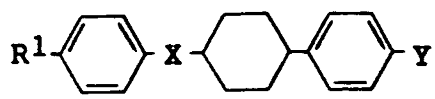

(Ib)

wherein $R^1$ is a normal or branched alkyl chain having 1 to 12 carbon atoms; X is a bridging group -COO- or -OCO, Y is a terminal group $-NO_2$ or -NCS.

**4.** The compound having the formula

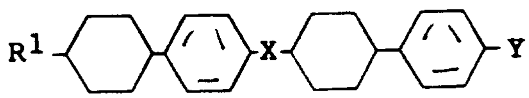

(Ic)

wherein $R^1$ is a normal or branched alkyl chain having 1 to 12 carbon atoms; X is a bridging group $-CHOHCH_2-$ or $-CH_2CH_2-$or $-CHOCH_3CH_2-$, Y is a terminal group $-NO_2$, $-NH_2$ or -NCS.

**5.** The compound having the formula

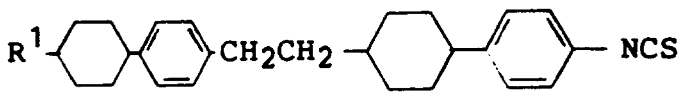

(Ic 4c)

wherein $R^1$ is a normal or branched alkyl chain having 1 to 12 carbon atoms.

**6.** The compound according to Claim 5 wherein $R^1$ is butyl, pentyl, hexyl or heptyl.

**7.** The compound having the formula

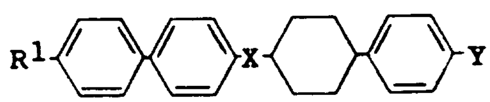

(Id)

wherein $R^1$ is normal or branched alkyl chain or a normal or branched alkoxy group having 1 to 12 carbon atoms; X is a bridging group $-CHOHCH_2-$, $-CH_2CH_2-$ or $-CHOCH_3CH_2-$, Y is a terminal group $-NO_2$, $-NH_2$ or -NCS.

**8.** The compound having the formula

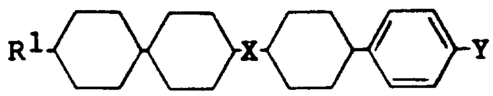

(Ie)

wherein $R^1$ is a normal or branched alkyl chain having 1 to 12 carbon atoms; X is a bridging group $-CHOHCH_2-$, $-CH_2CH_2$ or $-CHOCH_3CH_2-$, Y is a terminal group $-NO_2$, $-NH_2$ or -NCS.

**9.** A liquid crystalline mixture consisting of at least 2 components containing at least one component expressed by the formula

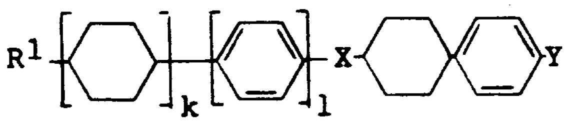

(I)

wherein $R^1$ is a normal or branched alkyl group $H_{2n+1}C_n$-or a normal or branched alkoxy group $H_{2n+1}C_nO$-, n being an integer from 1 to 12, X is a bridging group $-CHOHCH_2-$, $-CH_2CH_2-$, $-CHOCH_3CH_2-$, -COO-, -OCO-, Y is the terminal group -NCS, the cyclohexane rings are trans-1,4-disubstituted and the benzene rings are 1,4-disubstituted, in which the H-atom in 3- or 2-position can be replaced by a F-atom, k and l are integers 0, 1 or 2, under the condition that k + 1 = 2 or 3, besides if X denotes -COO- or -OCO-, then k + 1 may also be 1.

**10.** The liquid crystalline mixture according to Claim 9 wherein a component of formula I is used in a quantity of 5 to 50 wt.-%.

**11.** In a liquid crystalline mixture comprising 4-(trans-4-n-alkylcyclohexyl)benzeneisothiocyanates the improvement which results from introducing a compound of the formula

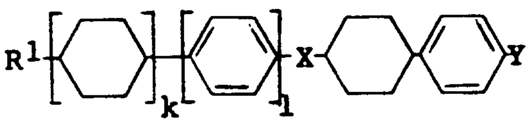

(I)

wherein $R^1$ is a normal or branched alkyl group $H_{2n+1}C_n$-or a normal or branched alkoxy group $H_{2n+1}C_nO$, n being an integer from 1 to 12, X is a bridging group $-CHOHCH_2-$, $-CH_2CH_2-$, $-CHOCH_3CH_2-$, -COO- or -OCO-, Y is the terminal group -NCS, the cyclohexane rings are trans-1,4-disubstituted and the benzene rings are 1,4-disubstituted, in which the H-atom in the 3- or 2-position can be replaced by a F-atom, k and l are integers 0, 1 or 2, under the condition that k + 1 = 2 or 3, besides if X denotes a -COO- or -OCO-group, then k + 1 may also be 1.

**12.** In an electrooptical cell comprising a liquid crystal dielectric disposed between two electrode plates, at least one of them is transparent, the improvement specific in that the applied liquid crystal dielectric is the mixture according to Claim 9.

**Patentansprüche**

**1.** Cyclohexylbenzolderivat der allgemeinen Formel

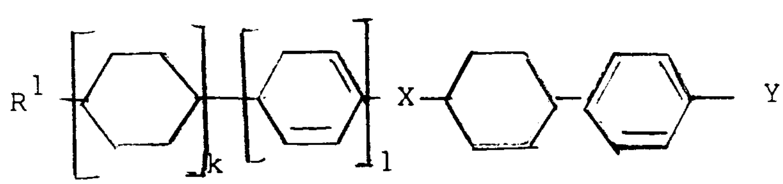

(I)

worin $R^1$ eine unverzweigte oder verzweigte $H_{2n+1}C_n$-Alkylgruppe oder eine unverzweigte oder verzweigte $H_{2n+1}C_nO$-Alkoxygruppe bedeutet, wobei n für eine ganze Zahl von 1 bis 12 steht, X für eine Brückengruppe $-CHOHCH_2-$, $-CH_2CH_2-$ oder $-CHOCH_3CH_2-$, -COO-, -OCO- und Y für eine endständige Gruppe $-NO_2$, $NH_2$ oder -NCS stehen, die Cyclohexanringe trans-1,4-disubstituiert und die Benzolringe 1,4-disubstituiert sind, wobei das H-Atom in 3- oder 2-Stellung durch ein F-Atom ersetzt sein kann, k und l ganze Zahlen 0, 1 oder 2 bedeuten, mit der Maßgabe, daß k + l 2 oder 3 ist, wobei, wenn X eine -COO- oder -OCO-Gruppe bedeutet, k + l auch 1 sein kann.

2. Verbindung der Formel

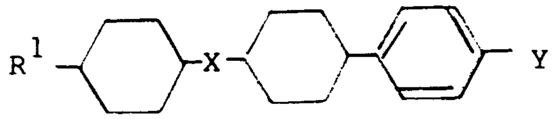

(Ia)

worin $R^1$ eine unverzweigte oder verzweigte Alkylkette mit 1 bis 12 C-Atomen bedeutet, X für eine Brückengruppe -COO- oder -OCO- und Y für eine endständige Gruppe $-NO_2$ oder -NCS stehen.

3. Verbindung der Formel

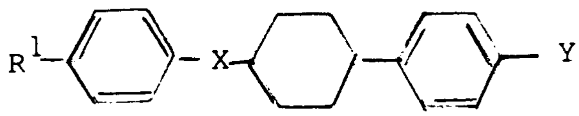

(Ib)

worin $R^1$ eine unverzweigte oder verzweigte Alkylkette mit 1 bis 12 C-Atomen bedeutet, X für eine Brückengruppe -COO- oder -OCO- und Y für eine endständige Gruppe $-NO_2$ oder -NCS stehen.

4. Verbindung der Formel

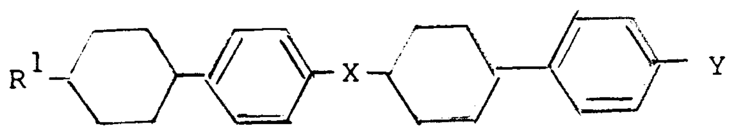

(Ic)

worin $R^1$ eine unverzweigte oder verzweigte Alkylkette mit 1 bis 12 C-Atomen bedeutet, X für eine Brückengruppe $-CHOHCH_2-$ oder $-CH_2CH_2-$ oder $-CHOCH_3CH_2-$ und Y für eine endständige Gruppe $-NO_2$, $-NH_2$ oder -NCS stehen.

5. Verbindung der Formel

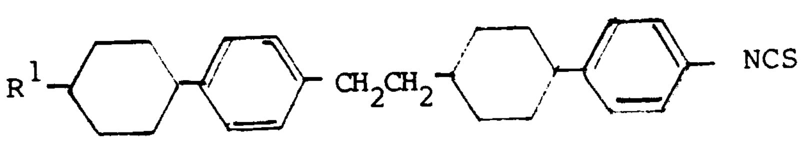

(Ic 4c)

worin $R^1$ eine unverzweigte oder verzweigte Alkylkette mit 1 bis 12 C-Atomen bedeutet.

6. Verbindung nach Anspruch 5, worin $R^1$ Butyl, Pentyl, Hexyl oder Heptyl bedeutet.

7. Verbindung der Formel

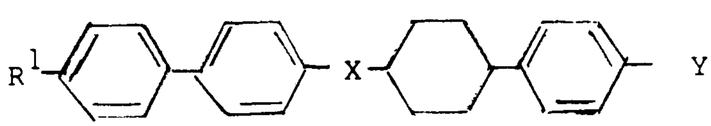

(Id)

worin $R^1$ eine unverzweigte oder verzweige Alkylkette oder eine unverzweigte oder verzweigte Alkoxygruppe mit 1 bis 12 C-Atomen bedeutet, X für eine Brückengruppe $-CHOHCH_2-$, $-CH_2CH_2-$ oder

$-CHOCH_3CH_2-$ und Y für eine endständige Gruppe $-NO_2$, $-NH_2$ oder -NCS stehen.

**8.** Verbindung der Formel

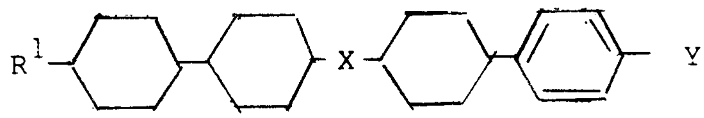

(Ie)

worin $R^1$ eine unverzweigte oder verzweigte Alkylkett mit 1 bis 12 C-Atomen bedeutet, X für eine Brückengruppe $-CHOHCH_2-$, $-CH_2CH_2$ oder $-CHOCH_3CH_2-$ und Y für eine endständige Gruppe $-NO_2$, $-NH_2$ oder -NCS stehen.

**9.** Flüssigkristallgemisch, bestehend aus wenigstens 2 Komponenten, die wenigstens eine Komponente der Formel

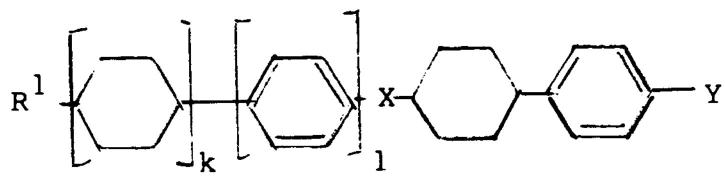

(I)

enthalten, worin $R^1$ eine unverzweigte oder verzweigte $H_{2n+1}C_n$-Alkylgruppe oder eine unverzweigte oder verzweigte $H_{2n+1}C_nO$-Alkoxygruppe bedeutet, wobei n für eine ganze Zahl von 1 bis 12 steht, X für eine Brückengruppe $CHOHCH_2-$, $-CH_2CH_2$, $-CHOCH_3CH_2-$, -COO- oder -OCO- und Y für die endständige Gruppe -NCS stehen, die Cyclohexanringe trans-1,4-disubstituiert und die Benzolringe 1,4-disubstituiert sind, wobei das H-Atom in 3- oder 2-Stellung durch ein F-Atom ersetzt sein kann, k und l ganze Zahlen 0, 1 oder 2 bedeuten, mit der Maßgabe, daß k + l 2 oder 3 ist, wobei, wenn X eine -COO- oder -OCO-Gruppe bedeutet, k + l auch 1 sein kann.

**10.** Flüssigkristallgemisch nach Anspruch 9, worin eine Komponente der Formel I in einer Menge von 5 bis 50 Gew.-% verwendet wird.

**11.** Flüssigkristallgemisch, das 4-(Trans-4-n-alkylcyclohexyl)benzolisothiocyanate enthält, wobei die Verbesserung darin besteht, daß eine Verbindung der Formel

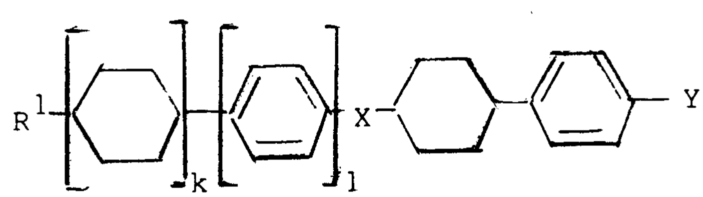

(I)

zugesetzt wird, worin $R^1$, eine unverzweigte oder verzweigte $H_{2n+1}C_n$- Alkylgruppe oder eine unverzweigte oder verzweigte $H_{2n+1}C_nO$-Alkoxygruppe bedeutet, wobei n für eine ganze Zahl von 1 bis 12 steht, X für eine Brückengruppe $-CHOHCH_2$, $-CH_2CH_2$, $-CHOCH_3CH_2$,-COO- oder -OCO- und Y für eine endständige Gruppe NCS stehen, die Cyclohexanringe trans-1,4-disubstituiert und die Benzolringe 1,4-disubstituiert sind, wobei das H-Atom in 3- oder 2-Stellung durch ein F-Atom ersetzt sein kann, k und l ganze Zahlen 0, 1 oder 2 bedeuten, mit der Maßgabe, daß k + l 2 oder 3 ist, wobei, wenn X eine -COO- oder -OCO-Gruppe bedeutet, k + l auch 1 sein kann.

**12.** Elektrooptische Zelle, die ein zwischen zwei Elektrodenplatten, von denen wenigstens eine transparent ist, angeordnetes Flüssigkristalldielektrikum umfaßt, wobei die Verbesserung darin besteht, daß das Flüssigkristalldielektrikum das Gemisch nach Anspruch 9 ist.

## Revendications

**1.** Dérivé du cyclohexylbenzène de formule générale

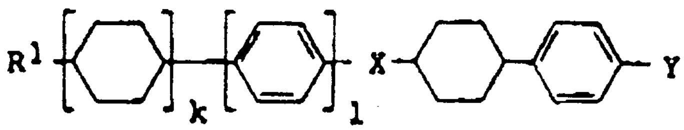

(I)

où $R^1$ est un groupement alkyle $H_{2n+1}C_n$- normal ou ramifié, ou un groupement alkoxy $H_{2n+1}C_nO$- normal ou ramifié, n étant un nombre entier entre 1 et 12 : X est un groupement pontant $-CHOHCH_2-$, $-CH_2CH_2-$ ou $-CHOCH_3CH_2-$; -COO-, -OCO-; Y est un groupement terminal $-NO_2$, $-NH_2$ ou -NCS ; les cycles cyclohexaniques sont des cycles trans-1,4-disubstitués et les cycles benzéniques sont 1,4-disubstitués, dans lesquels l'atome H en position 3 ou 2 peut être remplacé par un atome F ; k et l sont les nombres entiers 0, 1 ou 2, soumis à la condition que k+l = 2 ou 3, et qu'en outre, si X est un groupement -COO- ou -OCO-, k+l puisse aussi être égal à 1.

**2.** Composé ayant la formule

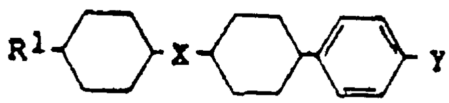

(Ia)

où $R^1$ est une chaîne alkyle normale ou ramifiée possédant de 1 à 12 atomes de carbone ; X est un groupement pontant -COO- ou -OCO-, Y est un groupement terminal $-NO_2$ ou -NCS.

**3.** Composé ayant la formule

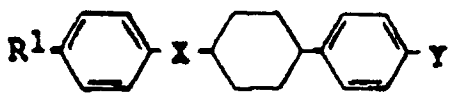

(Ib)

où $R^1$ est une chaîne alkyle normale ou ramifiée possédant de 1 à 12 atomes de carbone ; X est un groupement pontant -COO- ou -OCO-, Y est un groupement terminal $-NO_2$ ou -NCS.

**4.** Composé ayant la formule

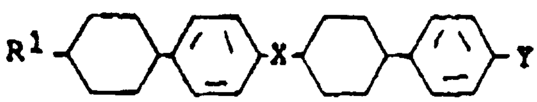

(Ic)

où $R^1$ est une chaîne alkyle normale ou ramifiée possédant de 1 à 12 atomes de carbone ; X est un groupement pontant $-CHOHCH_2-$ ou $-CH_2CH_2-$ou $-CHOCH_3CH_2-$, Y est un groupement terminal $-NO_2$, $-NH_2$ ou -NCS.

**5.** Composé ayant la formule

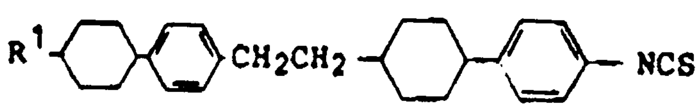

(Ic 4c)

où $R^1$ est une chaîne alkyle normale ou ramifiée possédant de 1 à 12 atomes de carbone.

**6.** Composé selon la Revendication 5 où $R^1$ est le radical butyle, pentyle, hexyle ou heptyle.

**7.** Composé ayant la formule

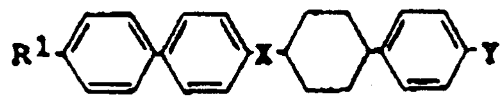

(Id)

où $R^1$ est une chaîne alkyle normale ou ramifiée ou un groupement alkoxy normal ou ramifié possédant de 1 à 12 atomes de carbone ; X est un groupement pontant $-CHOHCH_2-$, $-CH_2CH_2-$ ou $-CHOCH_3CH_2-$, Y est un groupement terminal $-NO_2$, $-NH_2$ ou -NCS.

**8.** Composé ayant la formule

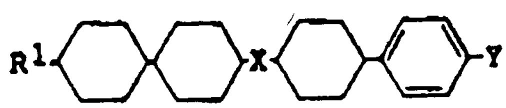

(Ie)

où $R^1$ est une chaîne alkyle normale ou ramifiée possédant de 1 à 12 atomes de carbone ; X est un groupement pontant $-CHOHCH_2-$, $-CH_2CH_2-$ ou $-CHOCH_3CH_2-$, Y est un groupement terminal-$NO_2$, $-NH_2$ ou -NCS.

**9.** Mélange cristallin liquide composé d'au moins deux constituants contenant au moins un constituant exprimé par la formule :

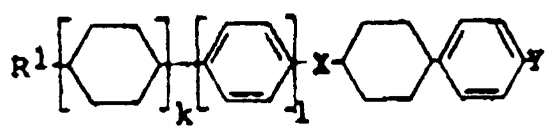

(I)

où $R^1$ est un groupement alkyle $H_{2n+1}C_n$- normal ou ramifié, ou un groupement alkoxy $H_{2n+1}C_nO$- normal ou ramifié, n étant un nombre entier entre 1 et 12 ; X est un groupement pontant $-CHOHCH_2-$, $-CH_2CH_2-$ ou $-CHOCH_3CH_2-$; -COO-, -OCO-; Y est le groupement terminal -NCS ; les cycles cyclohexaniques sont *trans*-1,4-disubstitués et les cycles benzéniques sont 1,4-disubstitués, dans lesquels l'atome H en position 3 ou 2 peut être remplacé par un atome F ; k et l sont les nombres entiers 0, 1 ou 2, soumis à la condition que $k+l = 2$ ou 3, et qu'en outre, si X est un groupement -COO- ou -OCO-, $k+l$ puisse alors aussi être égal à 1.

**10.** Mélange cristallin liquide selon la Revendication 9, où un constituant de formule I est utilisé dans une proportion de 5 à 50 % en poids.

**11.** Dans un mélange cristallin liquide comprenant des 4-(*trans*-4-n-alkylcyclohexyl)-benzèneisothiocyanates, le perfectionnement qui résulte de l'introduction d'un composé de formule

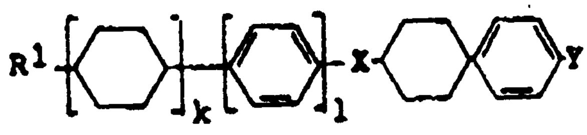

(I)

où $R^1$ est un groupement alkyle $H_{2n+1}C_n$- normal ou ramifié, ou un groupement alkoxy $H_{2n+1}C_nO$- normal ou ramifié, n étant un nombre entier entre 1 et 12 ; X est un groupement pontant $-CHOHCH_2-$, $-CH_2CH_2-$, $-CHOCH_3CH_2-$, -COO- ou -OCO- ; Y est le groupement terminal -NCS ; les cycles cyclohexaniques sont *trans*-1,4-disubstitués et les cycles benzéniques sont 1,4-disubstitués, dans lesquels l'atome H en position 3 ou 2 peut être remplacé par un atome F ; k et l sont les nombres

entiers 0, 1 ou 2, soumis à la condition que k+l = 2 ou 3, et qu'en outre, si X est un groupement -COO- ou -OCO-, k+l puisse alors aussi être égal à 1.

12. Dans une cellule électro-optique comprenant un diélectrique à cristaux liquides disposé entre deux lames d'électrodes, dont au moins une est transparente, le perfectionnement spécifique en ce que le diélectrique à cristaux liquides appliqué est le mélange selon la Revendication 9.